# EUROPEAN PATENT APPLICATION

(11) **EP 4 378 478 A1**
(43) Date of publication of application: **05.06.2024**
(21) Application number: 22849523.0
(22) Date of filing: 27.07.2022
(51) Int. Cl.: A61K 39/395, A61K 45/00, A61P 35/00, A61P 35/02, A61P 43/00, C07K 16/28, C07K 16/46, C12N 15/13, A61K 31/519

(54) **MEDICAMENT FOR TREATMENT AND/OR PREVENTION OF CANCER**

(30) Priority: 27.07.2021 JP 2021122072
(71) Applicant: Toray Industries, Inc., Tokyo 103-8666 (JP)
(72) Inventor: WASAI ,Ukei, Kamakura-shi, Kanagawa 248-8555 (JP); KUME, Masahiko, Kamakura-shi, Kanagawa 248-8555 (JP); OKANO, Fumiyoshi, Kamakura-shi, Kanagawa 248-8555 (JP); SAITO, Takanori, Kamakura-shi, Kanagawa 248-8555 (JP)
(74) Representative: Mewburn Ellis LLP
(86) International application number: PCT/JP2022/028878
(87) International publication number: WO 2023/008462

(57) **Abstract**

The present invention relates to a medicament for treatment and/or prevention of cancer, comprising an antibody or a fragment thereof having an immunological reactivity with CAPRIN-1 protein, and a MAPK pathway inhibitor together or separately in combination.

## Description

### Technical Field

The present invention relates to a medicament for treatment and/or prevention of a cancer, comprising an antibody against CAPRIN-1 protein, or a fragment thereof, and a MAPK pathway inhibitor.

### Background Art

Various antibody medicines targeting specific antigen proteins on cancer cells are applied as therapeutic agents for cancers with fewer side effects to cancer treatment because of their cancer specificity. For example, cytoplasmic-activation and proliferation-associated protein 1 (CAPRIN-1) is expressed on cell membrane surfaces of many solid cancers. Antibodies against this CAPRIN-1 protein are known to be promising in pharmaceutical uses for treatment and/or prevention of cancers (Patent Literature 1).

In recent years, treatment methods using combinations of pluralities of therapeutic agents for cancer have been clinically used as standard treatment methods in order to enhance the effectiveness of the therapeutic agents for cancers. In general, for example, breast cancer is treated by a treatment method using a combination of doxorubicin and cyclophosphamide or using a combination of a plurality of anticancer agents such as paclitaxel, trastuzumab, and pertuzumab. Therapeutic agents for cancers comprising anti-CAPRIN-1 antibodies as active ingredients have also been confirmed to have therapeutic effects on the cancers by combinations with chemotherapeutics (Patent Literature 2). However, treatment of a cancer by a combination of chemotherapeutics is not effective for every cancer to which the treatment is applied, and few combinations of chemotherapeutics synergistically drastically enhance therapeutic effects, though some combinations additively enhance therapeutic effects.

MAPK (mitogen-activated protein kinase) signaling is a signaling pathway that plays a central role in the control of cell proliferation, differentiation, and survival, and this pathway is constituted by four types of proteins RAS/RAF/MEK/ERK (Non Patent Literature 1). RAS activates RAF, and the activated RAF functions as MAP kinase kinase kinase (MAPKKK or MAP3K). Then, the RAF phosphorylates and activates MAP kinase kinase (MAPKK). This MAP kinase kinase (MAPKK) in this pathway is called MEK (MAPK or ERK kinase). MEK phosphorylates and activates the third and final enzyme in this pathway, i.e., MAP kinase (MAPK) called ERK (extracellular signal-regulated kinase). The ERK, when activated, can be translocated into the nucleus, and the ERK in this nucleus phosphorylates a transcriptional factor, thereby controlling its activity in an important cell process such as gene control, cell cycle transition or cell differentiation (Non Patent Literature 2).

MAPK signaling is important for physiological functions of normal cells, whereas its abnormal activation or appearance of mutant proteins are closely related to the onset or progression of cancers and approximately 85% of all cancers reportedly have abnormal MAPK signaling (Non Patent Literature 1). Against this backdrop, the MAPK signaling is regarded as an important target for cancer treatment. For example, an inhibitor targeting MEK located downstream in the MAPK signaling has been put into practical use as an anticancer agent (Non Patent Literature 1). However, a problem of the MEK inhibitor is that cancer cells acquire unresponsiveness to the inhibitor due to further abnormalities in the constituent factors of the MAPK signaling, activation of other signaling pathways that bypass the MAPK signaling, or the like so that the MEK inhibitor alone fails to completely cure the cancer, though high response is exhibited at an early stage from the start of treatment (Non Patent Literature 3). Likewise, although a RAF inhibitor exhibits marked clinical activity in patients with BRAF V600E or BRAF V600K melanoma, the drug efficacy of the RAF inhibitor alone is limited due to the emergence of drug resistance (Non Patent Literature 4).

### Citation List

### Patent Literature

Patent Literature 1: WO2010/016526
Patent Literature 2: WO2011/096535

### Non Patent Literature

Non Patent Literature 1: J. Hematol. Oncol., 2020, 17; 13(1): 113
Non Patent Literature 2: Biol Cell., 2001, 93(1-2): 53-62.
Non Patent Literature 3: Int. J. Mol. Sci., 2020, 21(3): 1102
Non Patent Literature 4: Nat Med. 2013, 19(11): 1401-9.

### Summary of Invention

### Object to be Achieved

An object of the present invention is to provide a medicament for treatment and/or prevention of a cancer specifically expressing CAPRIN-1 protein on a cell surface.

### Solution to Achieve Object

As a result of intensive studies, the present inventors have found that a combination (combined use) of an antibody against CAPRIN-1 protein, or a fragment thereof, having an immunological reactivity with cancer cells, and a MAPK pathway inhibitor exerts a very strong antitumor effect. On the basis of these findings, the present invention has been completed.

Specifically, the present invention relates to the following embodiments (1) to (18):
(1) A medicament for treatment and/or prevention of cancer, comprising an antibody or a fragment thereof having an immunological reactivity with CAPRIN-1 protein, and a MAPK (mitogen-activated protein kinase) pathway inhibitor together or separately in combination.
(2) The medicament according to (1), wherein the MAPK pathway inhibitor comprises a MEK inhibitor, a RAS inhibitor, a RAF inhibitor, an ERK inhibitor, or a combination of any two or more thereof.
(3) The medicament according to (2), wherein the MEK inhibitor is at least one selected from the group consisting of trametinib, cobimetinib, selumetinib, refametinib, SL-327, and their derivatives.
(4) The medicament according to (2), wherein the RAS inhibitor is at least one selected from the group consisting of sotorasib, lonafarnib, salirasib, and their derivatives.
(5) The medicament according to (2), wherein the RAF inhibitor is at least one selected from the group consisting of dabrafenib, ZM 336372, and their derivatives.
(6) The medicament according to (2), wherein the ERK inhibitor is at least one selected from the group consisting of bosutinib, SCH772984, and their derivatives.
(7) The medicament according to any of (1) to (6), wherein the antibody or the fragment has an immunological reactivity with CAPRIN-1 protein having an amino acid sequence shown by any one of the even numbered SEQ ID NOs: 2 to 30, or an amino acid sequence having 80% or more sequence identity with the amino acid sequence.
(8) The medicament according to any of (1) to (7), wherein the antibody or the fragment thereof has an immunological reactivity with an extracellular region of CAPRIN-1 protein present on a cancer cell surface.
(9) The medicament according to any of (1) to (8), wherein the antibody or the fragment thereof has an immunological reactivity with a partial polypeptide of CAPRIN-1 protein, the partial polypeptide having an amino acid sequence shown by any one of SEQ ID NOs: 31 to 35, 296 to 299, 308 and 309, or an amino acid sequence having 80% or more sequence identity with the amino acid sequence.
(10) The medicament according to any of (1) to (9), wherein the antibody is a monoclonal antibody or a polyclonal antibody.
(11) The medicament according to any of (1) to (10), wherein the antibody or the fragment thereof is any one of the following (A) to (M):
   (A) an antibody or a fragment thereof comprising a heavy-chain variable region comprising complementarity determining regions of SEQ ID NOs: 36, 37 and 38 (CDR1, CDR2 and CDR3, respectively) and a light-chain variable region comprising complementarity determining regions of SEQ ID NOs: 40, 41 and 42 (CDR1, CDR2 and CDR3, respectively), and having an immunological reactivity with CAPRIN-1 protein;
   (B) an antibody or a fragment thereof comprising a heavy-chain variable region comprising complementarity determining regions of SEQ ID NOs: 44, 45 and 46 (CDR1, CDR2 and CDR3, respectively) and a light-chain variable region comprising complementarity determining regions of SEQ ID NOs: 48, 49 and 50 (CDR1, CDR2 and CDR3, respectively), and having an immunological reactivity with CAPRIN-1 protein;
   (C) an antibody or a fragment thereof comprising a heavy-chain variable region comprising complementarity determining regions of SEQ ID NOs: 52, 53 and 54 (CDR1, CDR2 and CDR3, respectively) and a light-chain variable region comprising complementarity determining regions of SEQ ID NOs: 56, 57 and 58 (CDR1, CDR2 and CDR3, respectively), and having an immunological reactivity with CAPRIN-1 protein;
   (D) an antibody or a fragment thereof comprising a heavy-chain variable region comprising complementarity determining regions of SEQ ID NOs: 60, 61 and 62 (CDR1, CDR2 and CDR3, respectively) and a light-chain variable region comprising complementarity determining regions of SEQ ID NOs: 64, 65 and 66 (CDR1, CDR2 and CDR3, respectively), and having an immunological reactivity with CAPRIN-1 protein;
   (E) an antibody or a fragment thereof comprising a heavy-chain variable region comprising complementarity determining regions of SEQ ID NOs: 170, 171 and 172 (CDR1, CDR2 and CDR3, respectively) and a light-chain variable region comprising complementarity determining regions of SEQ ID NOs: 173, 174 and 175 (CDR1, CDR2 and CDR3, respectively), and having an immunological reactivity with CAPRIN-1 protein;
   (F) an antibody or a fragment thereof comprising a heavy-chain variable region comprising complementarity determining regions of SEQ ID NOs: 176, 177 and 178 (CDR1, CDR2 and CDR3, respectively) and a light-chain variable region comprising complementarity determining regions of SEQ ID NOs: 179, 180 and 181 (CDR1, CDR2 and CDR3, respectively), and having an immunological reactivity with CAPRIN-1 protein;
   (G) an antibody or a fragment thereof comprising a heavy-chain variable region comprising complementarity determining regions of SEQ ID NOs: 182, 183 and 184 (CDR1, CDR2 and CDR3, respectively) and a light-chain variable region comprising complementarity determining regions of SEQ ID NOs: 185, 186 and 187 (CDR1, CDR2 and CDR3, respectively), and having an immunological reactivity with CAPRIN-1 protein;
   (H) an antibody or a fragment thereof comprising a heavy-chain variable region comprising complementarity determining regions of SEQ ID NOs: 188, 189 and 190 (CDR1, CDR2 and CDR3, respectively) and a light-chain variable region comprising complementarity determining regions of SEQ ID NOs: 191, 192 and 193 (CDR1, CDR2 and CDR3, respectively), and having an immunological reactivity with CAPRIN-1 protein;
   (I) an antibody or a fragment thereof comprising a heavy-chain variable region comprising complementarity determining regions of SEQ ID NOs: 146, 147 and 148 (CDR1, CDR2 and CDR3, respectively) and a light-chain variable region comprising complementarity determining regions of SEQ ID NOs: 149, 150 and 151 (CDR1, CDR2 and CDR3, respectively), and having an immunological reactivity with CAPRIN-1 protein;
   (J) an antibody or a fragment thereof comprising a heavy-chain variable region comprising complementarity determining regions of SEQ ID NOs: 272, 273 and 274 (CDR1, CDR2 and CDR3, respectively) and a light-chain variable region comprising complementarity determining regions of SEQ ID NOs: 275, 276 and 277 (CDR1, CDR2 and CDR3, respectively), and having an immunological reactivity with CAPRIN-1 protein;
   (K) an antibody or a fragment thereof comprising a heavy-chain variable region comprising complementarity determining regions of SEQ ID NOs: 290, 291 and 292 (CDR1, CDR2 and CDR3, respectively) and a light-chain variable region comprising complementarity determining regions of SEQ ID NOs: 293, 294 and 295 (CDR1, CDR2 and CDR3, respectively), and having an immunological reactivity with CAPRIN-1 protein;
   (L) an antibody or a fragment thereof comprising a heavy-chain variable region comprising complementarity determining regions of SEQ ID NOs: 301, 302 and 303 (CDR1, CDR2 and CDR3, respectively) and a light-chain variable region comprising complementarity determining regions of SEQ ID NOs: 305, 306 and 307 (CDR1, CDR2 and CDR3, respectively), and having an immunological reactivity with CAPRIN-1 protein; and
   (M) an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein.
(12) The medicament according to any of (1) to (11), wherein the antibody or the fragment thereof is any one of the following (a) to (al):
   (a) an antibody or a fragment thereof comprising a heavy-chain variable region comprising the amino acid sequence of SEQ ID NO: 39 and a light-chain variable region comprising the amino acid sequence of SEQ ID NO: 43;
   (b) an antibody or a fragment thereof comprising a heavy-chain variable region comprising the amino acid sequence of SEQ ID NO: 47 and a light-chain variable region comprising the amino acid sequence of SEQ ID NO: 51;
   (c) an antibody or a fragment thereof comprising a heavy-chain variable region comprising the amino acid sequence of SEQ ID NO: 55 and a light-chain variable region comprising the amino acid sequence of SEQ ID NO: 59;
   (d) an antibody or a fragment thereof comprising a heavy-chain variable region comprising the amino acid sequence of SEQ ID NO: 63 and a light-chain variable region comprising the amino acid sequence of SEQ ID NO: 67;
   (e) an antibody or a fragment thereof comprising a heavy-chain variable region comprising the amino acid sequence of SEQ ID NO: 68 and a light-chain variable region comprising the amino acid sequence of SEQ ID NO: 69;
   (f) an antibody or a fragment thereof comprising a heavy-chain variable region comprising the amino acid sequence of SEQ ID NO: 70 and a light-chain variable region comprising the amino acid sequence of SEQ ID NO: 71;
   (g) an antibody or a fragment thereof comprising a heavy-chain variable region comprising the amino acid sequence of SEQ ID NO: 72 and a light-chain variable region comprising the amino acid sequence of SEQ ID NO: 73;
   (h) an antibody or a fragment thereof comprising a heavy-chain variable region comprising the amino acid sequence of SEQ ID NO: 74 and a light-chain variable region comprising the amino acid sequence of SEQ ID NO: 75;
   (i) an antibody or a fragment thereof comprising a heavy-chain variable region comprising the amino acid sequence of SEQ ID NO: 76 and a light-chain variable region comprising the amino acid sequence of SEQ ID NO: 77;
   (j) an antibody or a fragment thereof comprising a heavy-chain variable region comprising the amino acid sequence of SEQ ID NO: 78 and a light-chain variable region comprising the amino acid sequence of SEQ ID NO: 79;
   (k) an antibody or a fragment thereof comprising a heavy-chain variable region comprising the amino acid sequence of SEQ ID NO: 80 and a light-chain variable region comprising the amino acid sequence of SEQ ID NO: 81;
   (l) an antibody or a fragment thereof comprising a heavy-chain variable region comprising the amino acid sequence of SEQ ID NO: 82 and a light-chain variable region comprising the amino acid sequence of SEQ ID NO: 83;
   (m) an antibody or a fragment thereof comprising a heavy-chain variable region comprising the amino acid sequence of SEQ ID NO: 84 and a light-chain variable region comprising the amino acid sequence of SEQ ID NO: 85;
   (n) an antibody or a fragment thereof comprising a heavy-chain variable region comprising the amino acid sequence of SEQ ID NO: 86 and a light-chain variable region comprising the amino acid sequence of SEQ ID NO: 87;
   (o) an antibody or a fragment thereof comprising a heavy-chain variable region comprising the amino acid sequence of SEQ ID NO: 88 and a light-chain variable region comprising the amino acid sequence of SEQ ID NO: 89;
   (p) an antibody or a fragment thereof comprising a heavy-chain variable region comprising the amino acid sequence of SEQ ID NO: 90 and a light-chain variable region comprising the amino acid sequence of SEQ ID NO: 91;
   (q) an antibody or a fragment thereof comprising a heavy-chain variable region comprising the amino acid sequence of SEQ ID NO: 92 and a light-chain variable region comprising the amino acid sequence of SEQ ID NO: 93;
   (r) an antibody or a fragment thereof comprising a heavy-chain variable region comprising the amino acid sequence of SEQ ID NO: 94 and a light-chain variable region comprising the amino acid sequence of SEQ ID NO: 95;
   (s) an antibody or a fragment thereof comprising a heavy-chain variable region comprising the amino acid sequence of SEQ ID NO: 96 and a light-chain variable region comprising the amino acid sequence of SEQ ID NO: 97;
   (t) an antibody or a fragment thereof comprising a heavy-chain variable region comprising the amino acid sequence of SEQ ID NO: 98 and a light-chain variable region comprising the amino acid sequence of SEQ ID NO: 99;
   (u) an antibody or a fragment thereof comprising a heavy-chain variable region comprising the amino acid sequence of SEQ ID NO: 100 and a light-chain variable region comprising the amino acid sequence of SEQ ID NO: 101;
   (v) an antibody or a fragment thereof comprising a heavy-chain variable region comprising the amino acid sequence of SEQ ID NO: 102 and a light-chain variable region comprising the amino acid sequence of SEQ ID NO: 103;
   (w) an antibody or a fragment thereof comprising a heavy-chain variable region comprising the amino acid sequence of SEQ ID NO: 104 and a light-chain variable region comprising the amino acid sequence of SEQ ID NO: 105;
   (x) an antibody or a fragment thereof comprising a heavy-chain variable region comprising the amino acid sequence of SEQ ID NO: 106 and a light-chain variable region comprising the amino acid sequence of SEQ ID NO: 107;
   (y) an antibody or a fragment thereof comprising a heavy-chain variable region comprising the amino acid sequence of SEQ ID NO: 108 and a light-chain variable region comprising the amino acid sequence of SEQ ID NO: 109;
   (z) an antibody or a fragment thereof comprising a heavy-chain variable region comprising the amino acid sequence of SEQ ID NO: 110 and a light-chain variable region comprising the amino acid sequence of SEQ ID NO: 111;
   (aa) an antibody or a fragment thereof comprising a heavy-chain variable region comprising the amino acid sequence of SEQ ID NO: 112 and a light-chain variable region comprising the amino acid sequence of SEQ ID NO: 113;
   (ab) an antibody or a fragment thereof comprising a heavy-chain variable region comprising the amino acid sequence of SEQ ID NO: 114 and a light-chain variable region comprising the amino acid sequence of SEQ ID NO: 115;
   (ac) an antibody or a fragment thereof comprising a heavy-chain variable region comprising the amino acid sequence of SEQ ID NO: 116 and a light-chain variable region comprising the amino acid sequence of SEQ ID NO: 117;
   (ad) an antibody or a fragment thereof comprising a heavy-chain variable region comprising the amino acid sequence of SEQ ID NO: 118 and a light-chain variable region comprising the amino acid sequence of SEQ ID NO: 119;
   (ae) an antibody or a fragment thereof comprising a heavy-chain variable region comprising the amino acid sequence of SEQ ID NO: 120 and a light-chain variable region comprising the amino acid sequence of SEQ ID NO: 121;
   (af) an antibody or a fragment thereof comprising a heavy-chain variable region comprising the amino acid sequence of SEQ ID NO: 122 and a light-chain variable region comprising the amino acid sequence of SEQ ID NO: 123;
   (ag) an antibody or a fragment thereof comprising a heavy-chain variable region comprising the amino acid sequence of SEQ ID NO: 124 and a light-chain variable region comprising the amino acid sequence of SEQ ID NO: 125;
   (ah) an antibody or a fragment thereof comprising a heavy-chain variable region comprising the amino acid sequence of SEQ ID NO: 126 and a light-chain variable region comprising the amino acid sequence of SEQ ID NO: 127;
   (ai) an antibody or a fragment thereof comprising a heavy-chain variable region comprising the amino acid sequence of SEQ ID NO: 128 and a light-chain variable region comprising the amino acid sequence of SEQ ID NO: 129;
   (aj) an antibody or a fragment thereof comprising a heavy-chain variable region comprising the amino acid sequence of SEQ ID NO: 130 and a light-chain variable region comprising the amino acid sequence of SEQ ID NO: 131;
   (ak) an antibody or a fragment thereof comprising a heavy-chain variable region comprising the amino acid sequence of SEQ ID NO: 132 and a light-chain variable region comprising the amino acid sequence of SEQ ID NO: 133; and
   (al) an antibody or a fragment thereof comprising a heavy-chain variable region comprising the amino acid sequence of SEQ ID NO: 300 and a light-chain variable region comprising the amino acid sequence of SEQ ID NO: 304.
(13) The medicament according to any of (1) to (12), wherein the antibody is a human antibody, a humanized antibody, a chimeric antibody or a single chain antibody.
(14) The medicament according to any of (1) to (13), wherein the cancer is a cancer expressing CAPRIN-1 protein on a cell membrane surface.
(15) The medicament according to any of (1) to (14), wherein the cancer is melanoma, lung cancer, thyroid cancer, colon cancer, prostate cancer, ovarian cancer, pancreatic cancer, kidney cancer, breast cancer, gastric cancer, bile duct cancer, renal cell cancer, Hodgkin's lymphoma, head and neck cancer, mesothelioma, colorectal cancer, esophageal cancer, gastroesophageal cancer, hepatocellular cancer, glioblastoma, urothelial cancer, urinary bladder cancer, uterus cancer, primary central nervous system lymphoma, primary testicular lymphoma, biliary tract cancer, brain tumor, leukemia, lymphoma, liver cancer, sarcoma, fibrosarcoma, mastocytoma, adrenal cortex cancer, Ewing's tumor, multiple myeloma, testicle cancer, basal cell cancer, Paget's disease or skin cancer.
(16) An agent increasing drug efficacy of a pharmaceutical composition for treatment and/or prevention of cancer comprising an antibody or a fragment thereof having an immunological reactivity with CAPRIN-1 protein as an active ingredient, wherein the agent comprises MAPK pathway inhibitor as an active ingredient.
(17) An agent increasing drug efficacy of a pharmaceutical composition for treatment and/or prevention of cancer comprising MAPK pathway inhibitor as an active ingredient, wherein the agent comprises an antibody or a fragment thereof having an immunological reactivity with CAPRIN-1 protein as an active ingredient.
(18) A method for treating and/or preventing cancer, comprising administering an antibody or a fragment thereof having an immunological reactivity with CAPRIN-1 protein, and MAPK pathway inhibitor together or separately to a subject.

The present specification encompasses the contents disclosed in Japanese Patent Application No. 2021-122072 on which the priority of the present application is based.

### Advantageous Effects of Invention

The combination of an antibody against CAPRI-1 protein, or a fragment thereof and a MAPK pathway inhibitor according to the present invention exerts a stronger antitumor effect than that of the antibody against CAPRIN-1 protein alone and an existing chemotherapeutic alone. Thus, the combination of the antibody against CAPRIN-1 protein and the MAPK pathway inhibitor is effective for treatment or prevention of cancer.

### Brief Description of Drawings

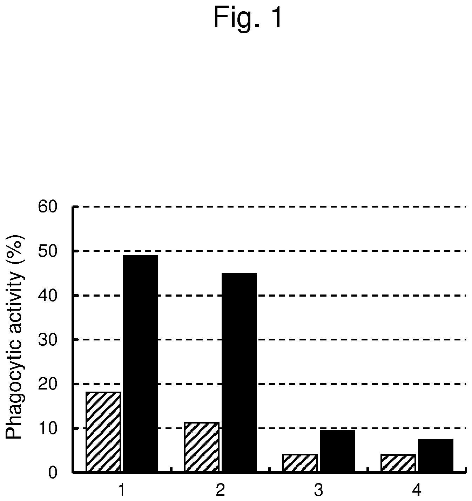

[Figure 1] Figure 1 is a diagram showing human monocytic cell (THP-1)-mediated phagocytic activity of a combination of an anti-CAPRIN-1 antibody and trametinib against a human cancer cell line. Reference number 1 depicts the phagocytic activity against a human melanoma cell line (A375), reference number 2 depicts the phagocytic activity against a human colon cancer cell line (HCT116), reference number 3 depicts the phagocytic activity against a human prostate cancer cell line (DU145), and reference number 4 depicts the phagocytic activity against a human lung cancer cell line (A549). Shaded bar graph: a test group without a drug used in combination (DMSO was added instead of the drug). Filled bar graph; a trametinib combination test group.

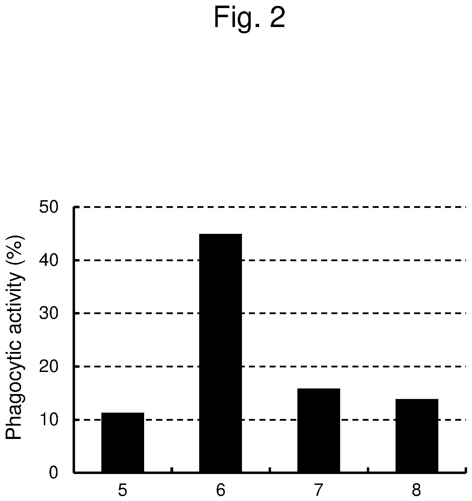

[Figure 2] Figure 2 is a diagram showing human monocytic cell (THP-1)-mediated phagocytic activity of a combination of an anti-CAPRIN-1 antibody and any of various drugs against a human colon cancer cell line HCT116. Reference number 5 depicts the phagocytic activity in a test group without a drug used in combination (DMSO was added instead of the drug to the cells), reference number 6 depicts the phagocytic activity in a trametinib combination test group (50 nM), reference number 7 depicts the phagocytic activity in a fluorouracil (5-FU) combination test group (0.4 µg/mL), and reference number 8 depicts the phagocytic activity in an irinotecan combination test group (15 µM).

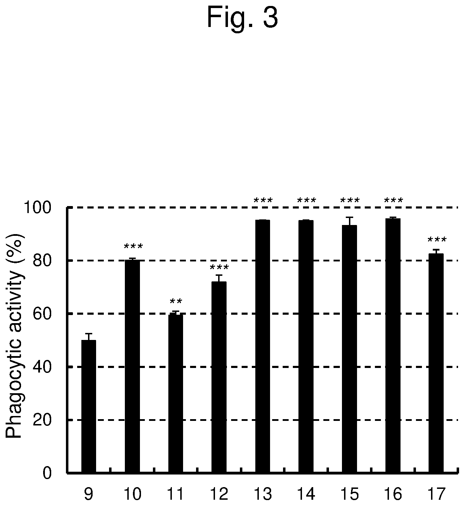

[Figure 3] Figure 3 is a diagram showing human monocytic cell (THP-1)-mediated phagocytic activity of a combination of an anti-CAPRIN-1 antibody and any of various drugs against a human colon cancer cell line HCT116. Reference number 9 depicts the phagocytic activity in a test group without a drug used in combination, reference number 10 depicts the phagocytic activity in a lonafarnib combination test group (10 µM), reference number 11 depicts the phagocytic activity in a sotorasib combination test group (10 µM), reference number 12 depicts the phagocytic activity in a salirasib combination test group (100 µM), reference number 13 depicts the phagocytic activity in a selumetinib combination test group (10 µM), reference number 14 depicts the phagocytic activity in a refametinib combination test group (1000 nM), reference number 15 depicts the phagocytic activity in a SL-327 combination test group (10 µM), reference number 16 depicts the phagocytic activity in a cobimetinib combination test group (800 nM), and reference number 17 depicts the phagocytic activity in a bosutinib combination test group (10 µM). Error bars depict standard deviation (S.D.). As a result of conducting Student's t test, the phagocytic activities of drug combination test groups were significantly higher than that of the test group without a drug used in combination (significance level: 5%). **, p < 0.01; ***, p < 0.001.

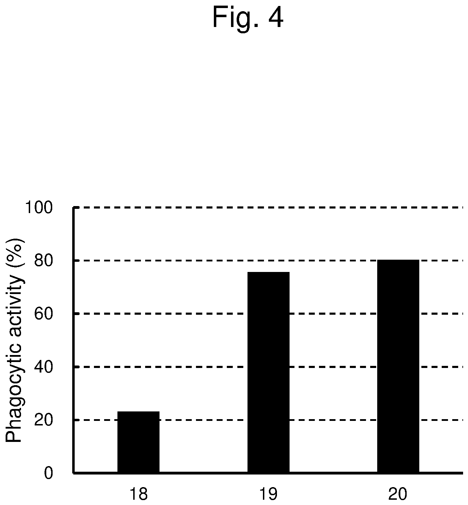

[Figure 4] Figure 4 is a diagram showing human monocytic cell (THP-1)-mediated phagocytic activity of a combination of an anti-CAPRIN-1 antibody and any of various drugs against a human colon cancer cell line HCT116. Reference number 18 depicts the phagocytic activity in a test group without a drug used in combination, reference number 19 depicts the phagocytic activity in a dabrafenib combination test group (10 µM), and reference number 20 depicts the phagocytic activity in a ZM 336372 combination test group (100 µM).

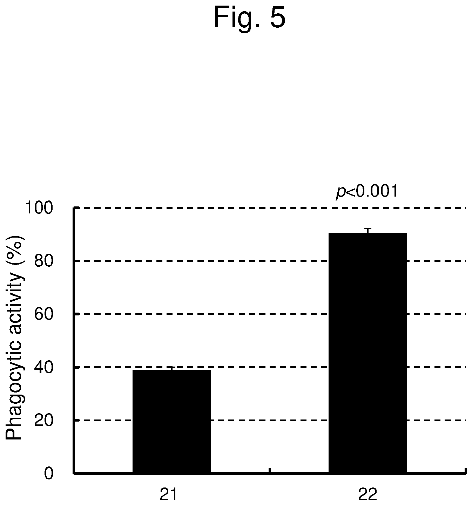

[Figure 5] Figure 5 is a diagram showing human monocytic cell (THP-1)-mediated phagocytic activity of a combination of an anti-CAPRIN-1 antibody and any of various drugs against a human colon cancer cell line HCT116. Reference number 21 depicts the phagocytic activity in a test group without a drug used in combination, and reference number 22 depicts the phagocytic activity in a SCH772984 combination test group (10 µM). Error bars depict standard deviation (S.D.). As a result of conducting Student's t test, the phagocytic activity of the SCH772984 combination test group was significantly higher than that of the test group without a drug used in combination (p < 0.001; significance level: 5%).

### Description of Embodiments

The antitumor effect of the combination of an antibody against CAPRIN-1 protein or a fragment thereof (hereinafter, referred to as an "anti-CAPRIN-1 antibody") and a MAPK pathway inhibitor, used in the present invention is preferably evaluated by examining *in vitro* the phagocytic activity against cancer cells by immunocytes in coculturing the cancer cells and the immunocytes as mentioned later. The immunocyte used in evaluating the antitumor effect *in vitro* may be any cell as long as the immunocyte is a blood cell having phagocytic activity, and preferably, is a human monocytic cell (THP-1 or U937). When an antibody binds to a cancer cell, this is recognized by an immunocyte so that the cancer cell is killed via phagocytic activity by the immunocyte. Therefore, an *in vivo* antitumor effect can be predicted by evaluating the *in vitro* antitumor effect.

The term "combination" (or "combined use") described herein refers to simultaneous administration or addition or administration or addition in a predetermined interval of the anti-CAPRIN-1 antibody and the MAPK pathway inhibitor as independent active ingredients to the same organism or cell. The interval may be simultaneous administration or may be 30 minutes later, 1 hour later, 3 hours later, 6 hours later, 12 hours later, 1 day later, 2 days later, 3 days later, 5 days later, 7 days later, 2 weeks later, 3 weeks later, or 4 weeks later. During one of the anti-CAPRIN-1 antibody or the MAPK pathway inhibitor exhibits its antitumor effect, the other can be administered or added.

The term "comprising ... together or separately in combination" described herein refers to comprising a plurality of drugs in a form that allows the drugs to be administered simultaneously or separately to a patient. The form may be, for example, the form of a so-called mixed formulation in which a plurality of drugs are mixed, or may be the form of a so-called kit formulation (pharmaceutical kit) comprising a plurality of drugs as individual formulations. The form also encompasses the form of a kit formulation comprising a plurality of drugs in any combination in two or more of formulations.

Such a kit formulation according to the present invention may be, for example, a kit formulation comprising: a formulation (or a pharmaceutical composition) comprising the anti-CAPRIN-1 antibody and a formulation (or a pharmaceutical composition) comprising the MAPK pathway inhibitor.

The anti-CAPRIN-1 antibody according to the present invention may be a monoclonal antibody or a polyclonal antibody and is preferably a monoclonal antibody. The antibody of the present invention may be any type of antibody as long as it can exhibit antitumor activity. The antibody may be a recombinant antibody, a human antibody, a humanized antibody, a chimeric antibody, or a non-human animal antibody.

Subjects being subjected to treatment and/or prevention of cancer according to the present invention are mammals such as primates, pet animals, livestock animals, or sport animals and preferably humans, dogs and cats, and more preferably humans.

Medicaments comprising an anti-CAPRIN-1 antibody and a MAPK route inhibiting agent as active ingredients, and methods for treating and/or preventing cancers, related to the present invention, will be explained below.

### <Anti-CAPRIN-1 antibody>

Among CAPRIN-1 proteins having an amino acid sequence shown by any one of the even numbered SEQ ID NOs: 2 to 30, which are specific examples of antigens having an immunological reactivity with the anti-CAPRIN-1 antibody used in the present invention, the amino acid sequences shown by SEQ ID NOs: 6, 8, 10, 12 and 14 are amino acid sequences of canine CAPRIN-1 proteins; the amino acid sequences shown by SEQ ID NOs: 2 and 4 are amino acid sequences of human CAPRIN-1 proteins; the amino acid sequence shown by SEQ ID NO: 16 is an amino acid sequence of a bovine CAPRIN-1 protein; the amino acid sequence shown by SEQ ID NO: 18 is an amino acid sequence of a horse CAPRIN-1 protein; the amino acid sequences shown by SEQ ID NOs: 20, 22, 24, 26 and 28 are amino acid sequences of mouse CAPRIN-1 proteins; and the amino acid sequence shown by SEQ ID NO: 30 is an amino acid sequence of a chicken CAPRIN-1 protein.

The anti-CAPRIN-1 antibody used in the present invention may have an immunological reactivity with a CAPRIN-1 protein variant having 80% or more, preferably 90% or more, more preferably 95% or more, and further preferably 99% or more sequence identity to the amino acid sequence shown by any one of the even numbered SEQ ID NOs: 2 to 30. The term "% sequence identity" as used herein means a percentage (%) of the number of identical amino acids (or nucleotides) to the total number of amino acids (or nucleotides) in the case that two sequences are aligned such that maximum similarity can be achieved with or without introduction of gaps.

In the present invention, the anti-CAPRIN-1 antibody refers to an antibody or a fragment (antigen binding fragment) thereof having an immunological reactivity with a full-length CAPRIN-1 protein or a fragment thereof. The term "immunological reactivity" used herein indicates the characteristics of an antibody specifically binding *in vivo* to a CAPRIN-1 protein or a partial polypeptide thereof.

The anti-CAPRIN-1 antibody used in the present invention may be a monoclonal antibody or a polyclonal antibody.

Polyclonal antibodies having an immunological reactivity with a full-length CAPRIN-1 protein or a fragment thereof (anti-CAPRIN-1 polyclonal antibodies) can be obtained, for example, in a manner described below. Serum is obtained after mice, human antibody-producing mice, rats, rabbits, chickens, or the like are immunized using a naturally occurring CAPRIN-1 protein or the protein fused with GST or the like, or a partial peptide thereof. The obtained serum is purified via ammonium sulfate precipitation, protein A, protein G, DEAE ion-exchange columns, affinity columns to which a CAPRIN-1 protein or a partial peptide is coupled, or the like.

Nucleotide sequences and amino acid sequences of CAPRIN-1 and homologs thereof used in the immunization can be obtained by, for example, accessing the website of GenBank (NCBI, USA) and using the BLAST or FASTA algorithm (Karlin and Altschul, Proc. Natl. Acad. Sci. USA, 90: 5873-5877, 1993; and Altschul et al., Nucleic Acids Res. 25: 3389-3402, 1997). Methods for producing CAPRIN-1 protein can be obtained with reference to WO2014/012479 or may employ cells or the like expressing CAPRIN-1 protein.

Monoclonal antibodies having an immunological reactivity with a full-length CAPRIN-1 protein or a fragment thereof (anti-CAPRIN-1 monoclonal antibodies) can be obtained, for example, in a manner described below. Breast cancer cells SK-BR-3 expressing CAPRIN-1, a full-length CAPRIN-1 protein or a fragment thereof, or the like is administered to mice for immunization. Splenocytes separated from the mice are fused with myeloma cells. Clones capable of producing anti-CAPRIN-1 monoclonal antibodies can be selected from the obtained fusion cells (hybridomas) to obtain these antibodies. The antibodies produced from the selected hybridomas can be obtained in the same way as the aforementioned method for purifying polyclonal antibodies.

The antibody used in the present invention includes human antibodies, humanized antibodies, chimeric antibodies, non-human animal antibodies, and single chain antibodies.

For human antibodies, human lymphocytes infected with EB virus are sensitized with a protein, protein-expressing cells, or a lysate thereof. The sensitized lymphocytes are fused with human-derived myeloma cells such as U266 cells. Antibodies having an immunological reactivity with a full-length CAPRIN-1 protein or a fragment thereof can be obtained from the obtained fusion cells.

A humanized antibody is a modified antibody, and it is sometimes referred to as a reshaped human antibody. It is known that a humanized antibody is constructed by transplanting complementarity determining regions of an immunized animal-derived antibody into complementarity determining regions of a human antibody. In addition, a general gene recombinant technique therefor is well known. Specifically, a DNA sequence designed in a manner that allows complementarity determining regions of mouse or rabbit antibody to be ligated to human antibody framework regions is synthesized by the PCR method using several oligonucleotides prepared in such a manner that the oligonucleotides have portions overlapping each other at an end of each thereof. A humanized antibody can be obtained by ligating the above obtained DNA to DNA encoding a human antibody constant region, incorporating the resultant into an expression vector, and introducing the vector into a host for antibody production (see EP-A-239400 and WO96/02576). Framework regions of human antibody ligated to each other via complementarity determining regions are selected on the assumption that complementarity determining regions can form a good antigen binding site. If necessary, amino acids in framework regions of an antibody variable region may be substituted in such a manner that complementarity determining regions in a reshaped human antibody form an appropriate antigen binding site (Sato K. et al., Cancer Research 1993, 53: 851-856). In addition, the framework regions may be substituted with framework regions from a different human antibody (see WO99/51743).

In general, antibodies are heteromultimeric glycoproteins each comprising at least two heavy chains and two light chains. Antibodies each comprise two identical light chains and two identical heavy chains. Each heavy chain has a heavy-chain variable region at one end thereof, to which some constant regions are bound in series. Each light chain has a light-chain variable region at one end thereof to which some constant regions are bound in series. Variable regions have specific variable regions, which are called complementarity determining regions (CDRs) and imparts binding specificity to an antibody. A relatively conserved portion in a variable region is called a framework region (FR). A complete heavy-chain or light-chain variable region comprises 4 FRs connected to each other via 3 CDRs (CDR1 to CDR3).

Sequences of human-derived heavy-chain and light-chain constant regions and variable regions can be obtained from, for example, NCBI (USA; GenBank, UniGene, etc.). For example, for a human IgG1 heavy-chain constant region, see registration No. J00228; for a human IgG2 heavy-chain constant region, see registration No. J00230; for a human light chain κ constant region, see sequences such as registration Nos. V00557, X64135, and X64133; and for a human light chain λ constant region, see sequences such as registration Nos. X64132 and X64134.

A chimeric antibody is an antibody produced by combining sequences from different animals. An example thereof is an antibody consisting of mouse antibody heavy-chain and light-chain variable regions and constant regions of human antibody heavy-chain and light-chain variable regions. Such a chimeric antibody can be produced by a known method. For example, it can be obtained by ligating DNA encoding an antibody V region to DNA encoding a human antibody C region, incorporating the resultant into an expression vector, and introducing the vector into a host for antibody production.

Non-human animal antibodies are obtained by immunizing non-human animals with sensitizing antigens according to a known method or by intraperitoneally, intracutaneously, or subcutaneously injecting sensitizing antigens into animals such as mice as a general method. For injecting sensitizing antigens, an appropriate amount of various adjuvants including CFA (Freund's complete adjuvant) is mixed therewith and the mixture is administered to animals several times. After immunization of animals and confirmation of an anti-CAPRIN-1 antibody contained in serum, the serum is obtained and the non-human animal antibody can be obtained by purification via ammonium sulfate precipitation, protein A, protein G, DEAE ion-exchange columns, affinity columns to which a CAPRIN-1 protein or a partial peptide thereof is coupled, or the like, as mentioned above. In the case of obtaining monoclonal antibodies from non-human animals, a monoclonal antibody is obtained by collecting immunocytes from the immunized animals and subjected to cell fusion with myeloma cells. The cell fusion of immunocytes with myeloma cells can be carried out according to a known method (see Kohler, G. and Milstein, C. Methods Enzymol. (1981) 73, 3-46).

The antibody used in the present invention can also be obtained as a gene recombinant antibody produced by cloning an antibody gene from a hybridoma, incorporating the clone into an adequate vector, introducing the vector into a host, and producing the antibody by using a gene recombinant technique (see Carl, A.K. Borrebaeck, James, W. Larrick, THERAPEUTIC MONOCLONAL ANTIBODIES, Published in the United Kingdom by MACMILLAN PUBLISHERS LTD, 1990).

Amino acids in a variable region (e.g., FR) or a constant region in the anti-CAPRIN-1 antibody used in the present invention may be substituted with different amino acids. The amino acid substitution is a substitution of 1 or more, for example, less than 15, less than 10, not more than 8, not more than 6, not more than 5, not more than 4, not more than 3, or not more than 2 amino acids, preferably 1 to 9 amino acids. A substituted antibody should have characteristics of specifically binding to the antigen and binding affinity for the antigen equivalent to or more than those of an unsubstituted antibody and should be an antibody that causes no rejection when applied to humans. The amino acid substitution is preferably a conservative amino acid substitution, which is a substitution between amino acids having similar characteristics in terms of charge, side chains, polarity, aromaticity, and the like. For example, characteristically similar amino acids can be classified into the following types: basic amino acids (arginine, lysine, and histidine); acidic amino acids (aspartic acid and glutamic acid); uncharged polar amino acids (glycine, asparagine, glutamine, serine, threonine, cysteine, and tyrosine); nonpolar amino acids (leucine, isoleucine, alanine, valine, proline, phenylalanine, tryptophan, and methionine); branched-chain amino acids (threonine, valine, isoleucine); and aromatic amino acids (phenylalanine, tyrosine, tryptophan, and histidine).

The anti-CAPRIN-1 antibody used in the present invention is expected to have a stronger antitumor effect when having higher binding affinity for CAPRIN-1 protein on cancer cell surfaces. Association constant (affinity constant) Ka (kon/koff) is preferably at least 10⁷ M⁻¹, at least 10⁸ M⁻¹, at least 5 × 10⁸ M⁻¹, at least 10⁹ M⁻¹, at least 5 × 10⁹ M⁻¹, at least 10¹⁰ M⁻¹, at least 5 × 10¹⁰ M⁻¹, at least 10¹¹ M⁻¹, at least 5 × 10¹¹ M⁻¹, at least 10¹² M⁻¹, or at least 10¹³ M⁻¹.

The anti-CAPRIN-1 antibody used in the present invention may be chemically modified. Examples of such an antibody modifier can include antibodies bound to various molecules such as polyethylene glycol (PEG) and antitumor compounds (for example, antitumor agents listed below). Regarding antibody modifiers of the present invention, substances that bind to an antibody are not limited. Such an antibody modifier can be obtained by chemically modifying an obtained antibody. Methods of such modification have been already established in the field related to the present invention.

The binding strength of the anti-CAPRIN-1 antibody used in the present invention against effector cells can be improved by substituting 1, 2 or several amino acids in the heavy-chain constant region of the antibody or by removing fucose bound to N-acetylglucosamine in a N-glycoside-linked sugar chain bound to the heavy-chain constant region. The anti-CAPRIN-1 antibody described above may have the amino acid substitution alone or may be a composition with an antibody bound to fucose.

Antibodies in which 1, 2 or several amino acids in the heavy-chain constant region have been substituted can be produced with reference to, for example, WO2004/063351, WO2011/120135, U.S. Patent No. 8388955, WO2011/005481, U.S. Patent No. 6737056, and WO2005/063351.

Antibodies in which fucose bound to N-acetylglucosamine in a N-glycoside-linked sugar chain in the heavy-chain constant region has been removed, or producing cells thereof can be produced with reference to U.S. Patent No. 6602684, EP Patent No. 1914244, and U.S. Patent No. 7579170. Compositions of antibodies in which fucose bound to N-acetylglucosamine in a N-glycoside-linked sugar chain bound to the heavy-chain constant region has been removed, with antibodies bound to fucose, or producing cells thereof can be produced with reference to, for example, U.S. Patent No. 8642292.

The anti-CAPRIN-1 polyclonal antibody and the anti-CAPRIN-1 monoclonal antibody used in the present invention, methods for producing or purifying antibodies and methods for producing a CAPRIN-1 protein or partial polypeptide thereof used in immunization can be obtained with reference to WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176 and WO2015/020212.

Specific examples of the anti-CAPRIN-1 antibody according to the present invention include anti-CAPRIN-1 antibodies described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176 and WO2015/020212 mentioned above. Preferred examples of the anti-CAPRIN-1 antibody include the following.

An antibody or a fragment thereof having an immunological reactivity with a partial polypeptide of a CAPRIN-1 protein having the amino acid sequence shown by SEQ ID NO: 2 or SEQ ID NO: 4 or an amino acid sequence having 80% or more (preferably 85% or more, more preferably 90% or more, further preferably 95% or more, and still further preferably 99% or more) sequence identity to the amino acid sequence.

An antibody or a fragment thereof having an immunological reactivity with a partial polypeptide of CAPRIN-1 protein, the partial polypeptide having the amino acid sequence shown by SEQ ID NO: 31 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 36, 37 and 38 (CDR1, CDR2 and CDR3, respectively) and a light-chain variable region comprising complementarity determining regions of SEQ ID NOs: 40, 41 and 42 (CDR1, CDR2 and CDR3, respectively), and having an immunological reactivity with CAPRIN-1 protein, an antibody or a fragment thereof comprising a heavy-chain variable region comprising complementarity determining regions of SEQ ID NOs: 140, 141 and 142 (CDR1, CDR2 and CDR3, respectively) and a light-chain variable region comprising complementarity determining regions of SEQ ID NOs: 143, 144 and 145 (CDR1, CDR2 and CDR3, respectively), and having an immunological reactivity with CAPRIN-1 protein, or an antibody or a fragment thereof comprising a heavy-chain variable region comprising complementarity determining regions of SEQ ID NOs: 164, 165 and 166 (CDR1, CDR2 and CDR3, respectively) and a light-chain variable region comprising complementarity determining regions of SEQ ID NOs: 167, 168 and 169 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 39 and a light-chain variable region comprising the amino acid sequence of SEQ ID NO: 43, an antibody or a fragment thereof comprising a heavy-chain variable region comprising the amino acid sequence of SEQ ID NO: 70 and a light-chain variable region comprising the amino acid sequence of SEQ ID NO: 71, or an antibody or a fragment thereof comprising a heavy-chain variable region comprising the amino acid sequence of SEQ ID NO: 78 and a light-chain variable region comprising the amino acid sequence of SEQ ID NO: 79.

An antibody or a fragment thereof having an immunological reactivity with a partial polypeptide of CAPRIN-1 protein, the partial polypeptide having the amino acid sequence shown by SEQ ID NO: 33 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 60, 61 and 62 (CDR1, CDR2 and CDR3, respectively) and a light-chain variable region comprising complementarity determining regions of SEQ ID NOs: 64, 65 and 66 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 63 and a light-chain variable region comprising the amino acid sequence of SEQ ID NO: 67.

An antibody or a fragment thereof having an immunological reactivity with a partial polypeptide of CAPRIN-1 protein, the partial polypeptide having the amino acid sequence shown by SEQ ID NO: 32 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 52, 53 and 54 (CDR1, CDR2 and CDR3, respectively) and a light-chain variable region comprising complementarity determining regions of SEQ ID NOs: 56, 57 and 58 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 55 and a light-chain variable region comprising the amino acid sequence of SEQ ID NO: 59.

An antibody or a fragment thereof having an immunological reactivity with a partial polypeptide of CAPRIN-1 protein, the partial polypeptide having the amino acid sequence shown by SEQ ID NO: 34 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 170, 171 and 172 (CDR1, CDR2 and CDR3, respectively) and a light-chain variable region comprising complementarity determining regions of SEQ ID NOs: 173, 174 and 175 (CDR1, CDR2 and CDR3, respectively), and having an immunological reactivity with CAPRIN-1 protein, or an antibody or a fragment thereof comprising a heavy-chain variable region comprising complementarity determining regions of SEQ ID NOs: 176, 177 and 178 (CDR1, CDR2 and CDR3, respectively) and a light-chain variable region comprising complementarity determining regions of SEQ ID NOs: 179, 180 and 181 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 80 and a light-chain variable region comprising the amino acid sequence of SEQ ID NO: 81, or an antibody or a fragment thereof comprising a heavy-chain variable region comprising the amino acid sequence of SEQ ID NO: 82 and a light-chain variable region comprising the amino acid sequence of SEQ ID NO: 83.

An antibody or a fragment thereof having an immunological reactivity with a partial polypeptide of CAPRIN-1 protein, the partial polypeptide having the amino acid sequence shown by SEQ ID NO: 35 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 182, 183 and 184 (CDR1, CDR2 and CDR3, respectively) and a light-chain variable region comprising complementarity determining regions of SEQ ID NOs: 185, 186 and 187 (CDR1, CDR2 and CDR3, respectively), and having an immunological reactivity with CAPRIN-1 protein, or an antibody or a fragment thereof comprising a heavy-chain variable region comprising complementarity determining regions of SEQ ID NOs: 188, 189 and 190 (CDR1, CDR2 and CDR3, respectively) and a light-chain variable region comprising complementarity determining regions of SEQ ID NOs: 191, 192 and 193 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 84 and a light-chain variable region comprising the amino acid sequence of SEQ ID NO: 85, or an antibody or a fragment thereof comprising a heavy-chain variable region comprising the amino acid sequence of SEQ ID NO: 86 and a light-chain variable region comprising the amino acid sequence of SEQ ID NO: 87.

An antibody or a fragment thereof comprising a heavy-chain variable region comprising complementarity determining regions of SEQ ID NOs: 44, 45 and 46 (CDR1, CDR2 and CDR3, respectively) and a light-chain variable region comprising complementarity determining regions of SEQ ID NOs: 48, 49 and 50 (CDR1, CDR2 and CDR3, respectively), and having an immunological reactivity with CAPRIN-1 protein, and preferably an antibody or a fragment thereof comprising a heavy-chain variable region comprising the amino acid sequence of SEQ ID NO: 47 and a light-chain variable region comprising the amino acid sequence of SEQ ID NO: 51.

An antibody or a fragment thereof having an immunological reactivity with a partial polypeptide of CAPRIN-1 protein, the partial polypeptide having the amino acid sequence shown by SEQ ID NO: 296 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 146, 147 and 148 (CDR1, CDR2 and CDR3, respectively) and a light-chain variable region comprising complementarity determining regions of SEQ ID NOs: 149, 150 and 151 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 72 and a light-chain variable region comprising the amino acid sequence of SEQ ID NO: 73.

An antibody or a fragment thereof having an immunological reactivity with a partial polypeptide of CAPRIN-1 protein, the partial polypeptide having the amino acid sequence shown by SEQ ID NO: 297 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 272, 273 and 274 (CDR1, CDR2 and CDR3, respectively) and a light-chain variable region comprising complementarity determining regions of SEQ ID NOs: 275, 276 and 277 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 114 and a light-chain variable region comprising the amino acid sequence of SEQ ID NO: 115.

An antibody or a fragment thereof having an immunological reactivity with a partial polypeptide of CAPRIN-1 protein, the partial polypeptide having the amino acid sequence shown by SEQ ID NO: 298 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 290, 291 and 292 (CDR1, CDR2 and CDR3, respectively) and a light-chain variable region comprising complementarity determining regions of SEQ ID NOs: 293, 294 and 295 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 120 and a light-chain variable region comprising the amino acid sequence of SEQ ID NO: 121.

An antibody or a fragment thereof having an immunological reactivity with a partial polypeptide of CAPRIN-1 protein, the partial polypeptide having the amino acid sequence shown by SEQ ID NO: 299 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 301, 302 and 303 (CDR1, CDR2 and CDR3, respectively) and a light-chain variable region comprising complementarity determining regions of SEQ ID NOs: 305, 306 and 307 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 300 and a light-chain variable region comprising the amino acid sequence of SEQ ID NO: 304.

An antibody or a fragment thereof having an immunological reactivity with a partial polypeptide of CAPRIN-1 protein, the partial polypeptide having the amino acid sequence shown by SEQ ID NO: 308 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 68 and a light-chain variable region comprising the amino acid sequence of SEQ ID NO: 69.

An antibody or a fragment thereof having an immunological reactivity with a partial polypeptide of CAPRIN-1 protein, the partial polypeptide having the amino acid sequence shown by SEQ ID NO: 309 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 68 and a light-chain variable region comprising the amino acid sequence of SEQ ID NO: 69.

In addition, the following anti-CAPRIN-1 antibodies are also preferably used.

An antibody or a fragment thereof comprising a heavy-chain variable region comprising the amino acid sequence of SEQ ID NO: 68 and a light-chain variable region comprising the amino acid sequence of SEQ ID NO: 69.

An antibody or a fragment thereof comprising a heavy-chain variable region comprising the amino acid sequence of SEQ ID NO: 70 and a light-chain variable region comprising the amino acid sequence of SEQ ID NO: 71.

An antibody or a fragment thereof comprising a heavy-chain variable region comprising the amino acid sequence of SEQ ID NO: 72 and a light-chain variable region comprising the amino acid sequence of SEQ ID NO: 73.

An antibody or a fragment thereof comprising a heavy-chain variable region comprising the amino acid sequence of SEQ ID NO: 74 and a light-chain variable region comprising the amino acid sequence of SEQ ID NO: 75.

An antibody or a fragment thereof comprising a heavy-chain variable region comprising the amino acid sequence of SEQ ID NO: 76 and a light-chain variable region comprising the amino acid sequence of SEQ ID NO: 77.

An antibody or a fragment thereof comprising a heavy-chain variable region comprising the amino acid sequence of SEQ ID NO: 78 and a light-chain variable region comprising the amino acid sequence of SEQ ID NO: 79.

An antibody or a fragment thereof comprising a heavy-chain variable region comprising the amino acid sequence of SEQ ID NO: 80 and a light-chain variable region comprising the amino acid sequence of SEQ ID NO: 81.

An antibody or a fragment thereof comprising a heavy-chain variable region comprising the amino acid sequence of SEQ ID NO: 82 and a light-chain variable region comprising the amino acid sequence of SEQ ID NO: 83.

An antibody or a fragment thereof comprising a heavy-chain variable region comprising the amino acid sequence of SEQ ID NO: 84 and a light-chain variable region comprising the amino acid sequence of SEQ ID NO: 85.

An antibody or a fragment thereof comprising a heavy-chain variable region comprising the amino acid sequence of SEQ ID NO: 86 and a light-chain variable region comprising the amino acid sequence of SEQ ID NO: 87.

An antibody or a fragment thereof comprising a heavy-chain variable region comprising the amino acid sequence of SEQ ID NO: 88 and a light-chain variable region comprising the amino acid sequence of SEQ ID NO: 89.

An antibody or a fragment thereof comprising a heavy-chain variable region comprising the amino acid sequence of SEQ ID NO: 90 and a light-chain variable region comprising the amino acid sequence of SEQ ID NO: 91.

An antibody or a fragment thereof comprising a heavy-chain variable region comprising the amino acid sequence of SEQ ID NO: 92 and a light-chain variable region comprising the amino acid sequence of SEQ ID NO: 93.

An antibody or a fragment thereof comprising a heavy-chain variable region comprising the amino acid sequence of SEQ ID NO: 94 and a light-chain variable region comprising the amino acid sequence of SEQ ID NO: 95.

An antibody or a fragment thereof comprising a heavy-chain variable region comprising the amino acid sequence of SEQ ID NO: 96 and a light-chain variable region comprising the amino acid sequence of SEQ ID NO: 97.

An antibody or a fragment thereof comprising a heavy-chain variable region comprising the amino acid sequence of SEQ ID NO: 98 and a light-chain variable region comprising the amino acid sequence of SEQ ID NO: 99.

An antibody or a fragment thereof comprising a heavy-chain variable region comprising the amino acid sequence of SEQ ID NO: 100 and a light-chain variable region comprising the amino acid sequence of SEQ ID NO: 101.

An antibody or a fragment thereof comprising a heavy-chain variable region comprising the amino acid sequence of SEQ ID NO: 102 and a light-chain variable region comprising the amino acid sequence of SEQ ID NO: 103.

An antibody or a fragment thereof comprising a heavy-chain variable region comprising the amino acid sequence of SEQ ID NO: 104 and a light-chain variable region comprising the amino acid sequence of SEQ ID NO: 105.

An antibody or a fragment thereof comprising a heavy-chain variable region comprising the amino acid sequence of SEQ ID NO: 106 and a light-chain variable region comprising the amino acid sequence of SEQ ID NO: 107.

An antibody or a fragment thereof comprising a heavy-chain variable region comprising the amino acid sequence of SEQ ID NO: 108 and a light-chain variable region comprising the amino acid sequence of SEQ ID NO: 109.

An antibody or a fragment thereof comprising a heavy-chain variable region comprising the amino acid sequence of SEQ ID NO: 110 and a light-chain variable region comprising the amino acid sequence of SEQ ID NO: 111.

An antibody or a fragment thereof comprising a heavy-chain variable region comprising the amino acid sequence of SEQ ID NO: 112 and a light-chain variable region comprising the amino acid sequence of SEQ ID NO: 113.

An antibody or a fragment thereof comprising a heavy-chain variable region comprising the amino acid sequence of SEQ ID NO: 114 and a light-chain variable region comprising the amino acid sequence of SEQ ID NO: 115.

An antibody or a fragment thereof comprising a heavy-chain variable region comprising the amino acid sequence of SEQ ID NO: 116 and a light-chain variable region comprising the amino acid sequence of SEQ ID NO: 117.

An antibody or a fragment thereof comprising a heavy-chain variable region comprising the amino acid sequence of SEQ ID NO: 118 and a light-chain variable region comprising the amino acid sequence of SEQ ID NO: 119.

An antibody or a fragment thereof comprising a heavy-chain variable region comprising the amino acid sequence of SEQ ID NO: 120 and a light-chain variable region comprising the amino acid sequence of SEQ ID NO: 121.

An antibody or a fragment thereof comprising a heavy-chain variable region comprising the amino acid sequence of SEQ ID NO: 122 and a light-chain variable region comprising the amino acid sequence of SEQ ID NO: 123.

An antibody or a fragment thereof comprising a heavy-chain variable region comprising the amino acid sequence of SEQ ID NO: 124 and a light-chain variable region comprising the amino acid sequence of SEQ ID NO: 125.

An antibody or a fragment thereof comprising a heavy-chain variable region comprising the amino acid sequence of SEQ ID NO: 126 and a light-chain variable region comprising the amino acid sequence of SEQ ID NO: 127.

An antibody or a fragment thereof comprising a heavy-chain variable region comprising the amino acid sequence of SEQ ID NO: 128 and a light-chain variable region comprising the amino acid sequence of SEQ ID NO: 129.

An antibody or a fragment thereof comprising a heavy-chain variable region comprising the amino acid sequence of SEQ ID NO: 130 and a light-chain variable region comprising the amino acid sequence of SEQ ID NO: 131.

An antibody or a fragment thereof comprising a heavy-chain variable region comprising the amino acid sequence of SEQ ID NO: 132 and a light-chain variable region comprising the amino acid sequence of SEQ ID NO: 133.

An antibody or a fragment thereof comprising a heavy-chain variable region comprising the amino acid sequence of SEQ ID NO: 300 and a light-chain variable region comprising the amino acid sequence of SEQ ID NO: 304.

In Examples mentioned later, the polyclonal antibody or the monoclonal antibody against a full-length CAPRIN-1 protein or a polypeptide of a portion of a region expressed on cell membrane surfaces of cancer cells was confirmed to exhibit reactivity with cell membrane surfaces of a plurality of human cancer cells. Furthermore, results indicating the response in human cancer patients were obtained, and a marked antitumor effect was obtained by which a tumor completely disappeared at some cancer sites.

### <MAPK pathway inhibitor>

The MAPK pathway refers to a signal transduction pathway that involves the activation of mitogen-activated protein kinase (MAPK) in the RAS/RAF/MEK/ERK pathway and links intracellular response to the binding of a growth factor to a cell surface receptor, and is also called MAPK signaling pathway, MAPK/ERK signal transduction pathway, RAF-MEK-ERK pathway, RAS/MAPK pathway, or RAS-RAF-MEK-ERK pathway in literatures. As a review of enzymes involved in the MAPK pathway, see, for example, Biochim Biophys Acta., 2007, 1773 (8): 1263-84. The term "MAPK pathway" is directed to its many protein components and a kinase cascade which is a portion of this signal transduction pathway as well as various targets controlled by this pathway.

The MAPK pathway inhibitor refers to an inhibitor for the biological activity of wild-type or any mutant of any of enzymes involved in the MAPK pathway. Specific examples of the MAPK pathway inhibitor include, but are not limited to, a RAS inhibitor, a RAF inhibitor, a MEK inhibitor and an ERK inhibitor. The MAPK pathway inhibitor may be a therapeutically effective derivative of a substance known as the MAPK pathway inhibitor as long as it has MAPK pathway inhibitory action.

### <RAS inhibitor>

The RAS inhibitor is a drug having the action of inhibiting the activity of RAS, one of the proteins constituting the MAPK pathway. RAS has a molecular weight of 21 kDa and belongs to a large family as monomer GTPase. The RAS subfamilies constitutes the small G protein superfamily, which includes three classical RASs, HRAS, KRAS, and NRAS, as well as R-RAS, TC21 (R-RAS2), M-RAS (R-RAS3), Rap1A, Rap1B, Rap2A, Rap2B, RalA, and RalB. RAS shifts from an inactive GDP-bound state to an active GTP-bound state in response to various extracellular stimuli. This GDP/GTP exchange reaction is accelerated by GEF(s) (guanine-nucleotide exchange factors). The GTP-bound RAS returns to a GDP-bound form by endogenous GTPase. This reaction is accelerated by GTPase-activating proteins (GAPs). The activated GTP-bound RAS interacts with a wide range of downstream targets and activates downstream signaling. RAF kinase is known as one of the major targets. RAS is a cancer gene having a high mutation frequency whose mutation is detected in approximately 30% of cancer patients. In recent years, a drug specifically inhibiting the proliferation of some cancers having a KRAS gene mutation (KRAS G12C mutation) has been approved, and other RAS inhibitors are also under medicinal development.

Specific examples of the RAS inhibitor include adagrasib (MRTX849), garsorasib, lonafamib (SCH66336), salirasib, sotorasib (AMG510), sotorasib racemate, (-)-rasfonin, (Rac)-antineoplaston A10, 6H05 (TFA), antineoplaston A10, Antitumor agent-60, APS6-45, ARS-1620, ARS-853 (ARS853), ASP2453, AZD4625, BAY-293, BI-2852, BI-3406, CASIN, CMC2.24, Deltarasin, Deltarasin hydrochloride, Diazepinomicin, Farnesyl thiosalicylic acid, FTI-276 trifluoroacetate salt, FTI-277, FTI-277 hydrochloride, GDC-6036, GGTI-286, GGTI-286 hydrochloride, GGTI298 trifluoroacetate, JDQ-443 (WO2021/120890), K20, Kobe0065, Kobe2602, KRAS mutant protein inhibitor 1, L-744,832 Dihydrochloride, LC-2, Manumycin A, MCP110, ML 210, MLS-573151, MRTX0902, MRTX1133, MRTX1133 formic, MRTX-1257, MRTX849 ethoxypropanoic acid, MRTX-EX185, NSC-658497, Oncrasin-1, Pan-RAS-IN-1, PHT-7.3, PROTAC K-Ras Degrader-1, PROTAC SOS1 degrader-2, RAS GTPase inhibitor 1, RAS inhibitor Abd-7, Ras Inhibitory Peptide, Ras/Rac Transformation Blocker, SCH 51344, RAS/RAS-RAF-IN-1, Rasarfin, RM-018, RMC-0331, RTIL 13, SAH-SOS1A, SAH-SOS1A TFA, SCH 51344, SOS1-IN-10 (WO2022/017519; compound 8), SOS1-IN-11, SOS1-IN-12, SOS1-IN-13, SOS1-IN-3 (WO2019/122129A1; compound I-1), SOS1-IN-4 (WO2021/228028; example 65), SOS1-IN-5 (WO2021/203768; compound 4), SOS1-IN-6, SOS1-IN-7, SOS1-IN-8 (WO2022/017339; compound 2), SOS1-IN-9 (WO2022/028506; compound 302), XRP44X, KRpep-2d, KY1220, NAV-2729, zoledronic acid (ZOL 446), BQU57, CID-1067700, Fendiline hydrochloride, ARS-1323 (WO2015/054572), ARS-1630 (WO2015/054572), KRas G12C inhibitor 1 (US2018/0072723), KRas G12C inhibitor 2 (WO2021/118877), KRas G12C inhibitor 3 (US2018/0072723 A1), KRAS G12C inhibitor 13 (WO2018/143315A1; compound 30), KRAS G12C inhibitor 14 (WO2019/110751A1; compound 17), KRAS G12C inhibitor 15 (WO2019/110751; compound 22), KRAS G12C inhibitor 16 (WO2019/110751; compound 39), KRAS G12C inhibitor 17 (WO2019/110751A1; compound 82), KRAS G12C inhibitor 18, KRAS G12C inhibitor 19 (WO2021/118877), KRAS G12C inhibitor 20 (CN112694475A; example 1), KRAS G12C inhibitor 21 (WO2021/219090; example 7), KRAS G12C inhibitor 22 (WO2021/219072A1; example 120), KRAS G12C inhibitor 23 (WO2021/218939; compound 1), KRAS G12C inhibitor 24 (CN113563323A; compound 1), KRAS G12C inhibitor 25 (WO2021/216770; compound 3), KRAS G12C inhibitor 26 (WO2021/109737), KRAS G12C inhibitor 27 (WO2021/109737), KRAS G12C inhibitor 28 (WO2021/113595; Example 1), KRAS G12C inhibitor 29 (WO2021/252339), KRAS G12C inhibitor 30 (WO2021/252339A1; compound 2), KRAS G12C inhibitor 31 (WO2021/252339), KRAS G12C inhibitor 32, KRAS G12C inhibitor 33 (WO2021/244603A1; compound 1), KRAS G12C inhibitor 34 (WO2021/239058), KRAS G12C inhibitor 35 (CN112920183A; compound 3), KRAS G12C inhibitor 36 (WO2021/121367; compound 1-2), KRAS G12C inhibitor 37 (WO2018/143315; compound 65), KRAS G12C inhibitor 38 (WO2021/129820; compound 171), KRAS G12C inhibitor 39 (WO2019/099524; compound 494), KRas G12C inhibitor 4 (WO2020/233592; compound 2), KRAS G12C inhibitor 40 (WO2021/129824; compound 70), KRAS G12C inhibitor 41 (WO2021/129824; compound 121), KRAS G12C inhibitor 42 (WO2020/146613; compound 10), KRAS G12C inhibitor 43, KRAS G12C inhibitor 44, KRAS G12C inhibitor 45, KRAS G12C inhibitor 46, KRAS G12C inhibitor 47, KRAS G12C inhibitor 48, KRAS G12C inhibitor 49, KRAS G12C inhibitor 5, KRAS G12C inhibitor 50, KRAS G12C inhibitor 51, KRAS G12C inhibitor 52, KRAS G12C inhibitor 53, KRAS G12C inhibitor 54, KRAS G12C inhibitor 55, K-Ras G12C-IN-1, K-Ras G12C-IN-2, K-Ras G12C-IN-3, K-Ras G12C-IN-4, KRAS G12D inhibitor 1, KRAS G12D inhibitor 10 (WO2021/108683; compound 34), KRAS G12D inhibitor 11 (WO2021/108683; compound 52), KRAS G12D inhibitor 12 (WO2021/108683; compound 134), KRAS G12D inhibitor 13 (WO2021/108683; compound 142), KRAS G12D inhibitor 14, KRAS G12D inhibitor 15 (WO2022/042630; compound 243), KRAS G12D inhibitor 3 TFA (WO2022/002102; compound 146), KRAS G12D inhibitor 3 (WO2022/002102; compound 146), KRAS G12D inhibitor 5, KRAS G12D inhibitor 6 (WO2021/108683; compound 112), KRAS G12D inhibitor 7 (WO2021/108683; compound 114), KRAS G12D inhibitor 8 (WO2021/107160; compound 2), KRAS G12D inhibitor 9 (WO2021/108683; compound 20), KRAS inhibitor-10 (WO2021/005165), KRAS inhibitor-11, KRAS inhibitor-12, KRAS inhibitor-13, KRAS inhibitor-14, KRAS inhibitor-15, KRAS inhibitor-16, KRAS inhibitor-18, KRAS inhibitor-4, KRAS inhibitor-6 (WO2017/087528; compound A), KRAS inhibitor-7 (WO2017/087528; compound B), KRAS inhibitor-8 (WO2017/087528; compound C), KRAS inhibitor-9, K-Ras (G12C) inhibitor 12, K-Ras (G12C) inhibitor 6, K-Ras (G12C) inhibitor 9, K-Ras-IN-1 and their pharmaceutically acceptable (known) salts or (known) derivatives. The RAS inhibitor is preferably adagrasib, garsorasib, lonafarnib, salirasib, sotorasib, zoledronic acid (ZOL 446), antineoplaston A10, or a pharmaceutically acceptable (known) salt or (known) derivative thereof, and more preferably lonafarnib, salirasib, sotorasib, or a pharmaceutically acceptable (known) derivative thereof.

Lonafarnib inhibits farnesyltransferase which activates H-ras, K-ras-4B and N-ras. Lonafarnib has a CAS number represented by 193275-84-2, an IUPAC name of 4-[2-[4-[(2R)-6,15-dibromo-13-chloro-4-azatricyclo[9.4.0.0^{3,8}]pentadeca-1(11),3(8),4,6,12,14-hexaen-2-yl]piperidin-1-yl]-2-oxoethyl]piperidine-1-carboxamide, a molecular formula of C₂₇H₃₁Br₂C₁N₄O₂, and a molecular weight of 638.8.

Salirasib (also known as farnesylthiosalicylic acid or FTS) inhibits the methylation of RAS (active RAS). Salirasib has a CAS number represented by 162520-00-5, an IUPAC name of 2-[(2E,6E)-3,7,11-trimethyldodeca-2,6,10-trienyl]sulfanylbenzoic acid, a molecular formula of C₂₂H₃₀O₂S, and a molecular weight of 358.5.

Sotorasib inhibits KRAS G12C. Sotorasib has a CAS number represented by 2252403-56-6, an IUPAC name of 6-fluoro-7-(2-fluoro-6-hydroxyphenyl)-1-(4-methyl-2-propan-2-ylpyridin-3-yl)-4-[(2S)-2-methyl-4-prop-2-enoylpiperazin-1-yl]pyrido[2,3-d]pyrimidin-2-one, a molecular formula of C₃₀H₃₀F₂N₆O₃, and a molecular weight of 560.59.

### <RAF inhibitor>

The RAF inhibitor is a drug having the action of inhibiting the activity of RAF, one of the proteins constituting the MAPK pathway. RAF kinase is a family consisting of three serine/threonine kinases associated with retrovirus cancer genes, and is constituted by three members ARAF, BRAF, and CRAF (RAF1). BRAF has a structure central to signal transduction, called activation loop. When BRAF is activated, this loop usually contributes to the stabilization of an active form through the phosphorylation of a plurality of sites. A particular amino acid (residue position: 600) in this loop is a location of an oncogenic mutation. When the position of this loop is shifted from a position in a dormant state due to change in amino acid at this position from valine to glutamic acid (called V600E mutation wherein V is valine, and E is glutamic acid), the enzyme becomes always in an active state so that cancer cells can proliferate without undergoing any usual control. The BRAF mutation is found in various cancers including melanoma and colon cancer. A CRAF mutation is found in a plurality of cancer types, and its activation is known to participate in the resistance of malignant melanoma to BRAF inhibitors. The activation of the RAF kinase requires dephosphorylation, and phosphorylation by protein tyrosine kinase of the SRC family and other protein serine/threonine kinases, in addition to interaction with RAS-GTP, and also requires dimerization of RAF. A RAF dimer in an active state then phosphorylates and activates MEK.

Specific examples of the RAF inhibitor include agerafenib, agerafenib hydrochloride, belvarafenib (HM95573/GDC-5573/RG6185), dabrafenib (GSK2118436A/GSK2118436), encorafenib-13C,d3, naporafenib (LXH254), rineterkib, tovorafenib (MLN2480), vemurafenib (PLX4032/RG7204/RO5185426), (Z)-GW 5074, Antitumor agent-60, AZ 628, AZ304, BI-882370, B-Raf IN 1, B-Raf IN 2 (WO2021/116055), B-Raf IN 5, B-Raf IN 6, B-Raf IN 7, B-Raf IN 8, B-Raf IN 9, BRAF inhibitor (WO2011/103196; Compound P-0850), BRAF V600E/CRAF-IN-1, BRAF V600E/CRAF-IN-2, CCT196969, Doramapimod (BIRB 796), Encorafenib-13C,d3, GDC-0879, GNE-9815, GSK-114, GW 5074, HG6-64-1 (HMSL 10017-101-1, WO2011/090738; example 9 (XI-1)), L-779450, LXH254, LY3009120 (DP-4978), MCP110, ML786 dihydrochloride, Pan-RAF kinase inhibitor 1 (WO2021/110141; compound 16B), PLX-4720, PLX-4720-d7, PLX7904, PLX7922, PLX8394, PROTAC B-Raf degrader 1, Raf inhibitor 1, Raf inhibitor 1 dihydrochloride, Raf inhibitor 2 (EP1003721B1), RAF mutant-IN-1 (WO2019107987A1), RAF709, RAF-IN-1, RAS/RAS-RAF-IN-1, Ro 5126766 (CH5126766), RRD-251, SB-590885, SB-682330A, SHR902275, TAK-580 (MLN 2480/BIIB-024), TAK-632, TBAP-001 (WO2015/075483), ZM 336372 and their pharmaceutically acceptable (known) salts or (known) derivatives. The RAF inhibitor is preferably agerafenib, belvarafenib, dabrafenib, naporafenib, rineterkib, tovorafenib, vemurafenib, or a pharmaceutically acceptable (known) salt or (known) derivative thereof, and more preferably dabrafenib (GSK2118436A/GSK2118436), ZM 336372, or a pharmaceutically acceptable (known) derivative thereof.

Dabrafenib (GSK2118436A/GSK2118436) inhibits the kinase activity of BRAF mutants (V600E, V600K and V600D). Dabrafenib has a CAS number represented by 1195765-45-7, an IUPAC name of N-[3-[5-(2-aminopyrimidin-4-yl)-2-tert-butyl-1,3-thiazol-4-yl]-2-fluorophenyl]-2,6-difluorobenzenesulfonamide, a molecular formula of C₂₃H₂₀F₃N₅O₂S₂, and a molecular weight of 519.6. Dabrafenib is also used as a mesylate salt thereof, which has a CAS number represented by 1195768-06-9, an IUPAC name of N-[3-[5-(2-aminopyrimidin-4-yl)-2-tert-butyl-1,3-thiazol-4-yl]-2-fluorophenyl]-2,6-difluorobenzenesulfonamide;methanesulfonic acid, a molecular formula of C₂₃H₂₀F₃N₅O₂S₂CH₄SO₃, and a molecular weight of 615.7. Dabrafenib simply described herein is a generic name for a free form and a mesylate salt of dabrafenib.

ZM 336372 (also known as Zinc00581684) inhibits the kinase activity of CRAF. ZM 336372 has a CAS number represented by 208260-29-1, an IUPAC name of 3-(dimethylamino)-N-[3-[(4-hydroxybenzoyl)amino]-4-methylphenyl]benzamide, a molecular formula of C₂₃H₂₃N₃O₃, and a molecular weight of 389.4.

### <MEK inhibitor>

The MEK inhibitor is a drug having the action of inhibiting the activity of MEK (MAPK/ERK kinase), one of the proteins constituting the MAPK pathway. MEK is a bispecific kinase (enzyme having both serine/threonine kinase and tyrosine kinase activities) that is activated by phosphorylation downstream of RAF in the MAPK signaling pathway. A typical phosphorylation substrate of MEK is ERK (extracellular signal-regulated kinase), and one of the main actions of the MEK inhibitor is the suppression of ERK phosphorylation. ERK in an inactive state is typically localized in the cytoplasm, while translocated into the nucleus when activated by phosphorylation, and phosphorylates various transcriptional factors including c-Jun, c-Myc, c-Fos, ETS 1, and the like. ERK regulates the expression of many genes via such activity control of the transcriptional factors. MEK has a plurality of isoforms, among which, particularly, MEK1 and MEK2 play an important role in the development or progression of cancers and are responsible for signal transduction in the MAPK pathway. Therefore, the MEK inhibitor is preferably a MEK1 and/or MEK2 inhibitor.

Specific examples of the MEK inhibitor include trametinib (GSK1120212/JTP-74057), cobimetinib (GDC-0973/XL518), binimetinib (MEK162/ARRY-162/ARRY-438162), selumetinib (AZD6244/ARRY-142886), mirdametinib, pelitinib (EKB-569/AS703026/MSC1936369B/SAR 245509), refametinib (BAY 869766/RDEA119), zapnometinib (PD0184264/ATR-002), pimasertib (AS703026/MSC1936369B/SAR 245509), (S,R,S)-AHPC-Me-C10-Br, 1-Allylcyclopropane-1-sulfonyl Chloride, APS-2-79, APS-2-79 hydrochloride, APS-2-79 hydrochloride, Arctigenin, ARRY142866, ARRY438162, AZD6244, AZD8330 (ARRY-424704/ARRY-704), Balamapimod (MKI 833), BAY 869766, BI-847325, C16-PAF, CH4987655, CH5126766, CHMFL-EGFR-202, CI-1040 (PD 184352), CIF, CInQ-03, CKI27, CS-3006, E6201 (ER-806201), EBI-1051, Epiberberine chloride, G-573, GDC-0623 (RG 7421/MEK inhibitor 1), GDC0973, GSK1120212, Hypothemycin, Isorhamnetin, JTP-74057, Lidocaine, Lidocaine hydrochloride, Lidocaine-d10, Lidocaine-d10 hydrochloride, Lidocaine-d10 N-Oxide, MAP855, MEK Inhibitor Set, MEK Inhibitor VIII, MEK inhibitor, MEK/PI3K-IN-1, MEK/PI3K-IN-2, MEK1/2 Inhibitor IV, MEK1/2-IN-2, MEK162 (ARRY438162), MEK-IN-1 (WO2008/076415), MEK-IN-5, MS432, MSC-2015103B, MSC-1936369, Myricetin, PD 184352, PD 198306, PD0325901 (MEK1/2 Inhibitor III), PD0325901-O-C2-dioxolane, PD184161, PD318088, PD-334581, PD98059, RDEA 119, RG7167, RG7304, RG7420, RGB-286638, RGB-286638 free base, Ro 5126766 (CH5126766), RO4987655 (CH4987655), SHR-7390, SL-327, TAK-733, TCS PIM-1 1 (SC 204330), trans-Zeatin, trans-Zeatin-d5, U0124, U0126, U0126-EtOH, XL518 and their pharmaceutically acceptable (known) salts or (known) derivatives. The MEK inhibitor is preferably trametinib, cobimetinib, binimetinib, selumetinib, mirdametinib, pelitinib, refametinib, zapnometinib, pimasertib, SL-327, or a pharmaceutically acceptable (known) salt or (known) derivative thereof, and more preferably trametinib, cobimetinib, selumetinib, refametinib, SL-327, or a pharmaceutically acceptable (known) derivative thereof.

Trametinib (GSK1120212/JTP-74057) has a CAS number represented by 871700-17-3, an IUPAC name of N-[3-[3-cyclopropyl-5-(2-fluoro-4-iodoanilino)-6,8-dimethyl-2,4,7-trioxopyrido[4,3-d]pyrimidin-1-yl]phenyl]acetamide, a molecular formula of C₂₆H₂₃FIN₅O₄, and a molecular weight of 615.4. Trametinib is poorly soluble in itself and as such, is also used as DMSO solvate thereof. The trametinib DMSO solvate has a CAS number represented by 1187431-43-1, an IUPAC name of N-[3-[3-cyclopropyl-5-(2-fluoro-4-iodoanilino)-6,8-dimethyl-2,4,7-trioxopyrido[4,3-d]pyrimidin-1-yl]phenyl]acetamide;methylsulfinylmethane, a molecular formula of C₂₈H₂₉FIN₅O₅S, and a molecular weight of 693.5. Trametinib simply described herein is a generic name for a free form and DMSO solvate of trametinib.

Cobimetinib inhibits the kinase activity of MEK1 and MEK2 and has a CAS number represented by 934660-93-2, an IUPAC name of [3,4-difluoro-2-(2-fluoro-4-iodoanilino)phenyl]-[3-hydroxy-3-[(2S)-piperidin-2-yl]azetidin-1-yl]methanone, a molecular formula of C₂₁H₂₁F₃IN₃O₂, and a molecular weight of 531.3. Cobimetinib is also used as a fumarate salt thereof, which has a CAS number represented by 1369665-02-0, an IUPAC name of (E)-but-2-enedioic acid;[3,4-difluoro-2-(2-fluoro-4-iodoanilino)phenyl]-[3-hydroxy-3-[(2S)-piperidin-2-yl]azetidin-1-yl]methanone, a molecular formula of (C₂₁H₂₁F₃IN₃O₂)₂·C₄H₄O₄, and a molecular weight of 1178.7. Cobimetinib simply described herein is a generic name for a free form and fumarate salt of cobimetinib.

Selumetinib inhibits the kinase activity of MEK. Selumetinib has a CAS number represented by 606143-52-6, an IUPAC name of 6-(4-bromo-2-chloroanilino)-7-fluoro-N-(2-hydroxyethoxy)-3-methylbenzimidazole-5-carboxamide, a molecular formula of C₁₇H₁₅BrClFN₄O₃, and a molecular weight of 457.7. Selumetinib is also used as a sulfate salt thereof, which has a CAS number represented by 943332-08-9, an IUPAC name of 6-(4-bromo-2-chloroanilino)-7-fluoro-N-(2-hydroxyethoxy)-3-methylbenzimidazole-5-carboxamide;sulfuric acid, a molecular formula of C₁₇H₁₅BrClFN₄O₃·H₂SO₄, and a molecular weight of 555.8. Selumetinib simply described herein is a generic name for a free form and sulfate salt of selumetinib.

Refametinib inhibits the kinase activities of MEK1 and MEK2. Refametinib has a CAS number represented by 923032-37-5, an IUPAC name of N-[3,4-difluoro-2-(2-fluoro-4-iodoanilino)-6-methoxyphenyl]-1-[(2S)-2,3-dihydroxypropyl]cyclopropane-1-sulfonamide, a molecular formula of C₁₉H₂0F₃IN₂O₅S, and a molecular weight of 572.3.

SL-327 inhibits the kinase activities of MEK1 and MEK2. SL-327 has a CAS number represented by 305350-87-2, an IUPAC name of (Z)-3-amino-3-(4-aminophenyl)sulfanyl-2-[2-(trifluoromethyl)phenyl]prop-2-enenitrile, a molecular formula of C₁₆H₁₂F₃N₃S, and a molecular weight of 335.3.

### <ERK inhibitor>

The ERK inhibitor is a drug having the action of inhibiting the activity of ERK, one of the proteins constituting the MAPK signaling pathway. ERK (extracellular signal-regulated kinase) is a serine/threonine kinase and generally refers to isoforms of ERK1 of 44 kDa and ERK2 of 42 kDa. When low-molecular-weight G protein RAS is activated, RAF and MEK are sequentially activated. The activated MEK phosphorylates tyrosine and threonine in the activation loop of ERK so that the kinase activity of the ERK itself is turned on. This series of signal transduction events is a mainstream of the MAP kinase pathway and called RAS-RAF-MEK-ERK signal transduction cascade. The activated ERK is known to phosphorylate diverse substrates and thereby participate in the exertion of various cell functions such as cell proliferation, differentiation, or survival, cell motility, or new tumor formation.

Specific examples of the ERK inhibitor include tizaterkib (WO2017/080979A1), alobresib (GS-5829), bortezomib (PS-341), bosutinib (SKI-606), edaxeterkib, ravoxertinib (GDC-0994), rineterkib, temuterkib (LY3214996), ulixertinib (BVD-523), ERK1/2 inhibitor 4 (WO2020/238776), ERK1/2 inhibitor 5 (WO2020/238776), ERK1/2 inhibitor 6 (WO2021/063335; compound 1), ERK1/2 inhibitor 7 (WO2021/110168; WX006), ERK1/2 inhibitor 8 (WO2021/110168; WX007), ERK1/2 inhibitor 3 (WO2021/218912; compound 1), (E)-Osmundacetone, (rel)-AR234960, 2,5-Dihydroxyacetophenone, ACA-28, Adjudin, AG126, AKT-IN-11, Astragaloside IV, ASTX-029, AZD0364 (ATG-017), Bohemine, C16-PAF, Cafestol, CC-90003, Cearoin, Chicanine, CHPG, CHPG sodium salt, CKLF1-C27 TFA, CKLF1-C27, Corynoxeine, Cucurbitacin IIb, DEL-22379, Deltonin, DMU-212, EF24, Enniatin A1, Enniatin B, Enniatin B1, epi-Eriocalyxin A, ERK1/2 inhibitor 1, ERK1/2 inhibitor 2, ERK2 IN-1, ERK-IN-2, ERK-IN-3, ERK-IN-3 benzenesulfonate, FR 180204, Gypenoside L, HIOC, Hirsutenone, HI-TOPK-032, Honokiol, I-191, KO-947, Lidocaine, LM22B-10, Longdaysin, Loureirin B, Magnolin, Methylthiouracil, MK-8353 (SCH900353), Mogrol, Motixafortide (BL-8040), MRTX-1257, Mulberroside A, Nitidine chloride, NMDAR/TRPM4-IN-2 free base, Omtriptolide, Pachymic acid, Pamoic acid disodium, Pamoic acid, PD98059, Piperlongumine, Pluripotin (SC1), SCH772984, Tauroursodeoxycholate, TBHQ, TCS ERK 11e, Tenuifoliside A, TIC10 (ONC201), trans-Zeatin, trans-Zeatin-d5, Urolithin B, VX-11e, Withanolide B, Xantocillin, Yoda 1, ZM 241385 and their pharmaceutically acceptable (known) salts or (known) derivatives. The ERK inhibitor is preferably tizaterkib, alobresib, bortezomib, bosutinib, edaxeterkib, ravoxertinib, rineterkib, rineterkib hydrochloride, temuterkib, ulixertinib, SCH772984, or a pharmaceutically acceptable (known) salt or (known) derivative thereof, and more preferably, bosutinib, SCH772984, or a pharmaceutically acceptable (known) derivative thereof.

Bosutinib (SKI-606) is a Src/Abl inhibitor and also inhibits MAPK/ERK. Bosutinib has a CAS number represented by 380843-75-4, an IUPAC name of 4-(2,4-dichloro-5-methoxyanilino)-6-methoxy-7-[3-(4-methylpiperazin-1-yl)propoxy]quinoline-3-carbonitrile, a molecular formula of C₂₆H₂₉Cl₂N₅O₃, and a molecular weight of 530.4. Bosutinib is also used as a hydrate thereof, which has a CAS number represented by 918639-08-4, an IUPAC name of 4-(2,4-dichloro-5-methoxyanilino)-6-methoxy-7-[3-(4-methylpiperazin-1-yl)propoxy]quinoline-3-carbonitrile;hydratea, a molecular formula of C₂₆H₂₉Cl₂N₅O₃·H₂O, and a molecular weight of 548.5. Bosutinib simply described herein is a generic name for a free form and hydrate of bosutinib.

SCH772984 inhibits the kinase activity of ERK1/2 and has a CAS number represented by 942183-80-4, an IUPAC name of (3R)-1-[2-oxo-2-[4-(4-pyrimidin-2-ylphenyl)piperazin-1-yl]ethyl]-N-(3-pyridin-4-yl-lH-indazol-5-yl)pyrrolidine-3-carboxamide, a molecular formula of C₃₃H₃₃N₉O₂, and a molecular weight of 587.7.

### <Other drugs>

The medicament of the present invention may comprise, as an active ingredient, an antitumor agent known in literatures, etc., in addition to the anti-CAPRIN-1 antibody and the MAPK pathway inhibitor, as long as it does not inhibit the effects as the medicament of the present invention. Specific examples of known antitumor agents include, but are not particularly limited to, 5-fluorouracil, irinotecan, oxaliplatin, carboplatin, cisplatin, nedaplatin, gemcitabine, paclitaxel, nabpaclitaxel, imiquimod, immune checkpoint inhibitors, doxorubicin, daunorubicin, cyclophosphamide, methotrexate, thiotepa, busulfan, improsulfan, piposulfan, benzodopa, carboquone, meturedopa, uredopa, altretamine, triethylenemelamine, triethylenephosphoramide, triethilenethiophosphoramide, trimethylolomelamine, bullatacin, bullatacinone, camptothecin, bryostatin, callystatin, cryptophycin 1, cryptophycin 8, dolastatin, duocarmycin, eleutherobin, pancratistatin, sarcodictyin, spongistatin, chlorambucil, chloRNAphazine, cholophosphamide, estramustine, ifosfamide, mechlorethamine, mechlorethamine oxide hydrochloride, melphalan, novembichin, phenesterine, prednimustine, trofosfamide, uracil mustard, carmustine, chlorozotocin, fotemustine, lomustine, nimustine, ranimustine, calicheamicin, dynemicin, clodronate, esperamicin, aclacinomycin, actinomycin, authramycin, azaserine, bleomycin, cactinomycin, carabicin, carminomycin, carzinophilin, chromomycin, dactinomycin, detorbicin, 6-diazo-5-oxo-L-norleucine, adriamycin, epirubicin, esorubicin, idarubicin, marcellomycin, mitomycin C, mycophenolic acid, nogalamycin, olivomycin, peplomycin, potfiromycin, puromycin, quelamycin, rodorubicin, streptonigrin, streptozocin, tubercidin, ubenimex, zinostatin, zorubicin, denopterin, pteropterin, trimetrexate, fludarabine, 6-mercaptopurine, thiamiprine, thioguanine, ancitabine, azacitidine, 6-azauridine, carmofur, cytarabine, dideoxyuridine, doxifluridine, enocitabine, floxuridine, androgens such as calusterone, dromostanolone propionate, epitiostanol, mepitiostane, testolactone, aminoglutethimide, mitotane, trilostane, frolinic acid, aceglatone, aldophosphamide glycoside, aminolevulinic acid, eniluracil, amsacrine, bestrabucil, bisantrene, edatraxate, defofamine, demecolcine, diaziquone, elfomithine, elliptinium acetate, epothilone, etoglucid, lentinan, lonidamine, maytansine, ansamitocine, mitoguazone, mitoxantrone, mopidanmol, nitraerine, pentostatin, phenamet, pirarubicin, losoxantrone, podophyllinic acid, 2-ethylhydrazide, procarbazine, razoxane, rhizoxin, schizophyllan, spirogermanium, tenuazonic acid, triaziquone, roridine A, anguidine, urethane, vindesine, dacarbazine, mannomustine, mitobronitol, mitolactol, pipobroman, gacytosine, docetaxel, chlorambucil, gemcitabine, 6-thioguanine, mercaptopurine, vinblastine, etoposide, ifosfamide, mitoxantrone, vincristine, vinorelbine, novantrone, teniposide, edatrexate, daunomycin, aminopterin, xeloda, ibandronate, difluoromethylomithine (DMFO), topoisomerase inhibitors, retinoic acid, folinic acid, and their pharmaceutically acceptable (known) salts or (known) derivatives.

### <Antitumor effect of present invention>

The antitumor effect of the combination of the anti-CAPRIN-1 antibody and the MAPK pathway inhibitor according to the present invention can be evaluated *in vivo* or *in vitro.* The *in vivo* antitumor effect can be evaluated by administering the anti-CAPRIN-1 antibody and the MAPK pathway inhibitor to an organism having cancer, measuring the size of a tumor after the administration, and examining the size of the cancer over time. Also, the *in vivo* antitumor effect can be evaluated by examining a survival rate of organisms. Alternatively, the *in vivo* antitumor effect may be evaluated by examining the ability to produce cytokines or chemokines. The *in vivo* antitumor effect can be further evaluated by examining prevention of cancer, prevention of metastasis or prevention of recurrence.

The *in vitro* antitumor effect can be evaluated by examining the cytotoxic activity or phagocytic activity against cancer cells by immunocytes in coculturing the cancer cells and the immunocytes. Thus, the antitumor effect of the combination of the anti-CAPRIN-1 antibody and the MAPK pathway inhibitor can be evaluated by adding the anti-CAPRIN-1 antibody and the MAPK pathway inhibitor in combination to a coculture system of cancer cells and immunocytes, and examining the cytotoxic activity or phagocytic activity against the cancer cells by the immunocytes. The immunocyte for use here may be any cell as long as the immunocyte is a blood cell having cytotoxic activity or phagocytic activity. A human NK cell is preferred for evaluating the cytotoxic activity, and human monocytic cells (THP-1 or U937) are preferred for evaluating the phagocytic activity. When an antibody binds to a cancer cell, this is recognized by an immunocyte so that the cancer cell is killed via cytotoxic activity or phagocytic activity by the immunocytes. Therefore, an *in vivo* antitumor effect can be predicted by evaluating the *in vitro* antitumor effect.

An ability of an anti-CAPRIN-1 antibody used in the present invention to bind to CAPRIN-1 can be determined via binding assay using, for example, ELISA, a Western blot method, immunofluorescence, or flowcytometry analysis.

The combination of the anti-CAPRIN-1 antibody and the MAPK pathway inhibitor according to the present invention increases an *in vitro* antitumor effect as compared with the anti-CAPRIN-1 antibody alone. The rate of increase is, for example, 1.1 or more times, preferably 1.5 or more times, more preferably 2 or more times, and further preferably 3 or more times.

### <Medicament for treatment and/or prevention of cancer>

A medicament of the present invention is aimed at treating and/or preventing cancer. A cancer targeted by the medicament of the present invention is not particularly limited as long as it is a cancer (cell) expressing CAPRIN-1 protein, particularly, a cancer (cell) expressing CAPRIN-1 protein on a cell membrane surface.

The term "treatment" used herein refers to treatment of cancer based on an antitumor effect mentioned above. The term "prevention" used herein refers to not only prevention of development of cancer but prevention of metastasis or recurrence of cancer.

Both the terms "tumor" and "cancer" used herein refer to malignant neoplasm, and thus they are used in an exchangeable manner.

Cancer that can be a target in the present invention is any cancer as long as the cancer expresses CAPRIN-1 protein on a cell membrane surface. The cancer is preferably melanoma, lung cancer, thyroid cancer, colon cancer, prostate cancer, ovarian cancer, pancreatic cancer, kidney cancer, breast cancer, gastric cancer, bile duct cancer, renal cell cancer, Hodgkin's lymphoma, head and neck cancer, mesothelioma, colorectal cancer, esophageal cancer, gastroesophageal cancer, hepatocellular cancer, glioblastoma, urothelial cancer, urinary bladder cancer, uterus cancer, primary central nervous system lymphoma, primary testicular lymphoma, biliary tract cancer, brain tumor, leukemia, lymphoma, liver cancer, sarcoma, fibrosarcoma, mastocytoma, adrenal cortex cancer, Ewing's tumor, multiple myeloma, testicle cancer, basal cell cancer, Paget's disease or skin cancer, and more preferably melanoma, non-small cell lung cancer, thyroid cancer, colon cancer, or prostate cancer. These cancers may be primary cancers, metastatic cancers, cancers that have metastasized, cancers that have recurred, postoperative cancers, or unresectable cancers. These cancers may have a specific gene mutation. Examples of the gene mutation include BRAF gene mutations, KRAS gene mutations, and EGFR gene mutations. The term melanoma is often used interchangeably with malignant melanoma.

More specifically, examples of the cancer include, but are not limited to, for example, Bowen's disease, prickle cell cancer, extramammary Paget's disease, mycosis fungoides, Sezary's syndrome, cutaneous T/NK-cell lymphoma, T-cell leukemia or lymphoma having a lesion only in the skin, cutaneous B-cell lymphoma (indolent group), cutaneous T-cell lymphatic or mammary adenoma, complex mammary adenoma, malignant mixed tumor of mammary gland, intraductal papillary carcinoma of mammary gland, lung adenocarcinoma, squamous cell cancer, small cell cancer, large cell cancer, glioma which is a neuroepithelial tissue tumor, glioblastoma, neuroblastoma, ependymoma, neuronal tumor, neuroectodermal tumor, neurilemoma, neurofibromatosis, meningioma, chronic lymphocytic leukemia, lymphoma, alimentary lymphoma, gastrointestinal lymphoma, small to medium cell lymphoma, cecal cancer, ascending colon cancer, descending colon cancer, transverse colon cancer, sigmoid colon cancer, rectal cancer, ovarian epithelial cancer, germ cell tumor, interstitial cell tumor, pancreatic duct cancer, invasive pancreatic duct cancer, adenocarcinoma of pancreatic cancer, acinar cell cancer, adenosquamous cancer, giant cell tumor, intraductal papillary mucinous tumor, mucinous cystadenocarcinoma, pancreatoblastoma, pancreatic islet cell tumor, Frantz's tumor, serous cystadenocarcinoma, solid pseudopapillary cancer, gastrinoma, glucagonoma, insulinoma, multiple endocrine neoplasia type-1 (Wermer syndrome), nonfunctional islet cell tumor, somatostatinoma, VIPoma, uterine cervical cancer, uterine body cancer, fibrosarcoma, osteosarcoma, joint sarcoma, Ewing sarcoma, Wilms's tumor, hepatoblastoma, soft tissue sarcoma, acute leukemia, chronic leukemia, spinal cord tumor, malignant soft tissue tumor, tumors of teratoma group, and head and neck cancer including hypopharynx cancer, oropharynx cancer, tongue cancer, nasopharyngeal cancer, oral cavity cancer, lip cancer, sinus cancer, voice box cancer, cancer of the renal pelvis and ureter, urinary bladder cancer, urethra cancer, testicular tumor, malignant pleural mesothelioma, malignant bone tumor, uterine body cancer (postoperative chemotherapy, chemotherapy at the time of metastasis or relapse), and pediatric malignant solid tumor (rhabdomyosarcoma, neuroblastoma, hepatoblastoma, medulloblastoma, nephroblastoma, retinoblastoma, central nervous system germ cell tumor, and Ewing sarcoma family of tumors). The cancer also includes a palpable cancer, a subcutaneously existing cancer, an intracutaneously existing cancer, a superficial cancer, cancer existing in the dermis or cancer existing in a non-parenchymal organ, and a progressive cancer which originate from the cancers described above. The cancer also includes a palpable cancer, a subcutaneously existing cancer, an intracutaneously existing cancer, a superficial cancer, cancer existing in the dermis, and cancer existing in a non-parenchymal organ, which originate from the cancers described above and have metastasized and recurred.

A preferable subject (patient) that can be a target is a mammal and is, for example, a mammal including primates, pet animals, livestock animals, and sport animals. Humans, dogs and cats are particularly preferable.

A medicament of the present invention can be formulated by a method known to persons skilled in the art. For instance, the medicament of the present invention can be parenterally used in the form of a parenteral injection of: an aseptic solution comprising water or a pharmacologically acceptable non-water solution; or a suspension liquid. For each formulation or pharmaceutical composition in the medicament of the present invention, the active ingredient (at least one of the anti-CAPRIN-1 antibody and the MAPK pathway inhibitor) may be appropriately combined with, for example, a pharmacologically acceptable carrier, medium or additive; specifically, sterilized water, physiological saline, an isotonic solution, a buffer (buffer solution, etc.), plant oil, an oily solution, an antioxidant, a solubilizer, an emulsifier, a suspension, a surfactant, a stabilizer, a fragrance, an excipient, or a binder, and preferably, may be formulated by mixing with them in a unit dosage form required for a generally acceptable pharmaceutical formulation. An amount of an active ingredient in a formulation is determined such that an appropriate dosage within an indicated range can be achieved.

An aseptic composition for injection can be prepared in accordance with general formulation practice using a vehicle such as distilled water for injection. An aqueous solution for injection purposes includes, for example, physiological saline or isotonic solutions comprising glucose and other adjuvants such as D-sorbitol, D-mannose, D-mannitol, and sodium chloride. Such solution may be used with an appropriate dissolution aid. Such dissolution aid includes, for example, alcohols such as ethanol and polyalcohol, such as propylene glycol, polyethylene glycol, or nonion surfactants such as polysorbate 80(TM) and HCO-60. Oily liquid includes, for example, sesame oil or soybean oil. Such oily liquid may be used in combination with a dissolution aid such as benzyl benzoate or benzyl alcohol. In addition, it may be mixed with a buffering agent such as a phosphate buffer solution or a sodium acetate buffer solution, a soothing agent such as procaine hydrochloride, a stabilizer such as benzyl alcohol or phenol, or an antioxidant. In general, a formulated injection solution is introduced into an adequate ample.

The above pharmaceutical composition is orally or parenterally administered. Preferably, it is parenterally administered. Specifically, dosage forms include injectable agents, intranasally-administered agents, transpulmonarily-administered agents, and percutaneously-administered agents. For example, injectable agents can be systemically or locally administered via intravenous injection, intramuscular injection, intraperitoneal injection, subcutaneous injection, or intratumoral injection. The percutaneously-administered agents include, for example, agents called liniments and external medicines. The external medicines include, for example, solid agents, solutions, sprays, ointments, creams, and gels.

The administration method can be appropriately determined depending on age, weight, gender, and symptoms of a patient. A single dose of a pharmaceutical composition comprising at least one of the anti-CAPRIN-1 antibody and the MAPK pathway inhibitor can be selected within a range of, for example, 0.0001 mg to 1000 mg per kg of body weight as the amount of each active ingredient. Alternatively, the dose of respective active ingredients can be selected within a range of, for example, 0.001 to 100000 mg per patient's body, or 0.1 to 300 mg or 1 mg to 30 mg per kg of patient's body weight; however, it is not necessarily limited thereto. The dose and the administration method are changed depending on patient age, weight, gender, and symptoms. However, persons skilled in the art can appropriately select the dose and the method.

### <Administration method>

Treatment and/or prevention of cancer with a medicament for treatment and/or prevention of cancer of the present invention includes various modes, in addition to administration as a medicament mentioned above. For example, respective active ingredients in a medicament of the present invention can be administered simultaneously, concurrently, or individually in a staggered manner. As a specific example, active ingredients can be administered within a time interval up to approximately 3 weeks, i.e., the second active ingredient can be administered from immediately up to approximately 3 weeks after administration of the first active ingredient. These administrations may be carried out subsequently to a surgical procedure, or a surgical procedure may be carried out between the administrations of the first and second drugs. In addition, a medicament for treatment and/or prevention of cancer of the present invention may be administered according to a plurality of administration cycles. For example, in the case of carrying out simultaneous administration of respective active ingredients in the medicament for treatment and/or prevention of cancer of the present invention, a pharmaceutical composition comprising both active ingredients is administered once per approximately 2 days to approximately 3 weeks as one cycle. Then, this treatment cycle may be repeated, if necessary, according to the judgment of a physician in charge. Likewise, in the case of scheduling a formulation in a staggered manner, respective administration periods of individual agents are adjusted so as to span the same period. The interval between cycles can vary from 0 to 2 months. Respective doses of the active ingredients in the medicament for treatment and/or prevention of cancer of the present invention can be set in the same way as in the respective doses of the active ingredients in the pharmaceutical composition.

### <Pharmaceutical kit>

A medicament for treatment and/or prevention of cancer of the present invention may be in the form of a pharmaceutical kit. The pharmaceutical kit is a package for using active ingredients in the form of separate pharmaceutical compositions (formulations) in a method for treating and/or preventing cancer. The package may comprise an instruction for administering each of the active ingredients. The respective active ingredients in the pharmaceutical compositions for treatment and/or prevention of cancer contained in the pharmaceutical kit can be in the form of pharmaceutical compositions each formulated as described above such that the active ingredients can be administered together or separately. Further, the pharmaceutical kit comprises active ingredients in amounts sufficient for one or more doses such that the active ingredients can be administered according to the administration method described above.

### <Treatment and/or prevention method>

On the basis of the contents specifically described above, the present invention further provides a method for treating and/or preventing cancer, comprising administering the medicament of the present invention, or the anti-CAPRIN-1 antibody and the MAPK pathway inhibitor according to the present invention to a subject (patient). For example, the present invention further provides a method for treating and/or preventing cancer, comprising administering the medicament, etc. of the present invention to a subject (patient) having cancer or suspected of having cancer. In this embodiment, for example, the anti-CAPRIN-1 antibody (antibody or fragment thereof) and the MAPK pathway inhibitor according to the present invention, and an optional antitumor agent contained in the medicament can be administered simultaneously or separately to the subject (patient).

### Examples

The present invention is hereafter described in detail with reference to the following examples, although the scope of the present invention is not limited thereto.

### (Example 1) Production of anti-CAPRIN-1 antibody

One hundred (100) µg of a human CAPRIN-1 recombinant protein produced according to Example 3 of WO2010/016526 was mixed with a MPL+TDM adjuvant (Sigma) in an amount equivalent to that of the recombinant protein. The mixture was used as an antigen solution per mouse. The antigen solution was administered intraperitoneally to 6-week-old Balb/c mice (Japan SLC Inc.) and then further administered 3 times and 24 times every week for completion of immunization. A spleen was removed on day 3 after the final immunization and then ground between two sterilized glass slides. Spleen cells were obtained by repeating the following procedure three times: washing with PBS (-) (Nissui Corporation), centrifuging at 1500rpm for 10 minutes, and removing supernatant. The obtained spleen cells were mixed with mouse myeloma cell SP2/0 (purchased from ATCC) at a ratio of 10 : 1. The PEG solution prepared by mixing 200 µl of RPMI1640 medium containing 10% FBS heated at 37°C and 800 µl of PEG1500 (Boehringer) was added to the cells. The solution was incubated for 5 minutes for cell fusion. Centrifugation was performed at 1700 rpm for 5 minutes to remove supernatants. Cells were suspended in 150 ml of RPMI1640 medium (HAT selective medium) containing 15% FBS, to which 2% equivalent of HAT solution (Gibco) had been added, and then seeded onto fifteen 96-well plates (Nunc) at 100 µl per well. Cells were cultured for 7 days under conditions of 37°C and 5% CO₂, so that hybridomas resulting from fusion of spleen cells to myeloma cells were obtained. Hybridomas were selected using binding affinity to CAPRIN-1 protein of the antibody produced by the prepared hybridomas as an indicator. The CAPRIN-1 protein solution (1 µg/ml) was added at 100 µl per well of 96-well plates and then incubated at 4°C for 18 hours. After each well was washed 3 times with PBS-T, 0.5% Bovine Serum Albumin (BSA) solution (Sigma) was added at 400 µl per well, and then the plates were incubated at room temperature for 3 hours. The solution was removed and then each well was washed 3 times with 400 µl of PBS-T. Each culture supernatant of the hybridomas obtained above was added at 100 µl per well and then incubated at room temperature for 2 hours. After each well was washed 3 times with PBS-T, an HRP-labeled anti-mouse IgG (H+L) antibody (Invitrogen) diluted 5000-fold with PBS was added at 100 µl per well and then incubated at room temperature for 1 hour. After each well was washed 3 times with PBS-T, a TMB substrate solution (Thermo) was added at 100 µl per well and then incubated for 15-30 minutes, so that a color reaction was performed. After color development, 1 N sulfuric acid was added at 100 µl per well to stop the reaction. Absorbance at 450 nm and absorbance at 595 nm were measured using an absorption spectrometer. As a result, a plurality of hybridomas producing antibodies with high absorbances were selected. The selected hybridomas were added at 0.5 hybridomas per well of 96-well plates and then cultured. After 1 week, hybridomas forming single colonies in wells were observed. Cells in these wells were further cultured. Hybridomas were selected using binding affinity to CAPRIN-1 protein of the antibody produced by cloned hybridomas as an indicator. The CAPRIN-1 protein solution (1 µg/ml) was added at 100 µl per well of 96-well plates and then incubated at 4°C for 18 hours. Each well was washed 3 times with PBS-T, a 0.5% BSA solution was added at 400 µl per well, and then incubated at room temperature for 3 hours. The solution was removed and then each well was washed 3 times with 400 µl of PBS-T. Each culture supernatant of the hybridomas obtained above was added at 100 µl per well and then incubated at room temperature for 2 hours. Each well was washed 3 times with PBS-T, an HRP-labeled anti-mouse IgG (H+L) antibody (Invitrogen) diluted 5000-fold with PBS was added at 100 µl per well and then incubated at room temperature for 1 hour. Each well was washed 3 times with PBS-T, a TMB substrate solution (Thermo) was added at 100 µl per well and then incubated for 15-30 minutes, so that a color reaction was performed. After color development, 1 N sulfuric acid was added at 100 µl per well to stop the reaction. Absorbance at 450 nm and absorbance at 595 nm were measured using an absorption spectrometer. As a result, a plurality of mouse monoclonal antibodies exerting reactivity with CAPRIN-1 protein were obtained.

Reactivity of each monoclonal antibody with human cancer cells confirmed to express CAPRIN-1 protein on cell membrane surfaces was further confirmed by flow cytometry. A mouse IgG control antibody exhibiting no reactivity with the cancer cells was used as a negative control. As a result of confirmation, several monoclonal antibodies were obtained which had stronger fluorescence intensity against the cancer cells than that of the mouse IgG control antibody and reacted strongly with the cell membrane surfaces of the cancer cells expressing CAPRIN-1 on the cell membrane surfaces. From among them, a monoclonal antibody against CAPRIN-1 described in WO2013/125630, which was an antibody comprising the amino acid sequence of a heavy-chain variable region shown by SEQ ID NO: 114 and the amino acid sequence of a light-chain variable region shown by SEQ ID NO: 115, was selected as a monoclonal antibody exhibiting reactivity with CAPRIN-1 protein.

CDR1 to CDR3 of the heavy-chain variable region of the antibody selected were identified. A nucleotide sequence was designed so as to be able to express a heavy-chain variable region having framework regions comprising a human antibody sequence. This nucleotide sequence was inserted to a vector for mammalian expression having an insert of a human IgG1 heavy-chain constant region. Likewise, CDR1 to CDR3 of the light-chain variable region were identified. A nucleotide sequence was designed so as to be able to express a light-chain variable region having framework regions comprising a human antibody sequence. This nucleotide sequence was inserted to a vector for mammalian expression having an insert of a human IgG1 light-chain constant region. These two recombinant expression vectors were introduced to mammalian cells according to a general method and then a culture supernatant containing humanized monoclonal antibody #1 (humanized antibody #1) against CAPRIN-1 was obtained. The amino acid sequences of heavy chain CDR1, CDR2, and CDR3 of the humanized monoclonal antibody #1 are shown by SEQ ID NOs: 272, 273, and 274, respectively. The amino acid sequences of light chain CDR1, CDR2, and CDR3 of the humanized monoclonal antibody #1 are shown by SEQ ID NOs: 275, 276, and 277, respectively.

The obtained culture supernatant containing the obtained humanized anti-CAPRIN-1 monoclonal antibody #1 was purified using Hitrap Protein A Sepharose FF (GE Healthcare BioSciences) according to a general method, replaced with PBS(-), and filtered through a 0.22 µm filter (Millipore) for preparation of the filtrate.

The specific reactivity of the anti-CAPRIN-1 antibody to CAPRIN-1 protein was detected and confirmed by ELISA using CAPRIN-1 protein immobilized on a plate.

The reactivity of the anti-CAPRIN-1 antibody with cancer cells without permeation treatment of cell membranes was examined by flow cytometry to confirm that a portion of CAPRIN-1 was expressed on cell membrane surfaces of cancer cells as shown in Examples given below.

It was confirmed by flow cytometry that, against all of breast cancer cells (BT-474), colon cancer cells (HT-29 and HCT116), lung cancer cells (QG56, H1650, and A549), gastric cancer cells (NCI-N87), uterus cancer cells (HEC-1-A), prostate cancer cells (22Rv1 and DU145), pancreatic cancer cells (Panc10.5), liver cancer cells (Hep3B), ovarian cancer cells (SKOV3), kidney cancer cells (Caki-2), brain tumor cells (U-87MG), urinary bladder cancer cells (T24), esophageal cancer cells (OE33), leukemia cells (OCI-AML5), lymphoma cells (Ramos), gallbladder cancer cells (TGBC14TKB), fibrosarcoma cells (HT-1080), and melanoma cells (G-361 and A375), which are human cancer cells confirmed to express CAPRIN-1 gene, and mouse kidney cancer cells (Renca) and mouse breast cancer cells (4T1) confirmed to express CAPRIN-1 gene, the humanized antibody #1 had stronger fluorescence intensity than that of a human IgG control antibody and rabbit IgG antibody serving as negative controls exhibiting no reactivity with the cancer cells and reacted strongly with the cell membrane surfaces of the cancer cells expressing CAPRIN-1.

Likewise, the anti-CAPRIN-1 antibodies described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176 and WO2015/020212 were also confirmed to react strongly with the cancer cell membrane surfaces.

### (Example 2) In vitro antitumor effect of combination of anti-CAPRIN-1 antibody and trametinib

Antitumor effects of combinations of an anti-CAPRIN-1 antibody and a MEK inhibitor trametinib were evaluated *in vitro.* Antitumor effects of combinations of an anti-CAPRIN-1 antibody and an existing chemotherapeutic fluorouracil (5-FU) or irinotecan (CPT-11) were evaluated simultaneously therewith and compared with the antitumor effect of the combination of anti-CAPRIN-1 antibody and trametinib. The trametinib used was Trametinib (Cat. No. CS-0060) purchased from ChemScene LLC.

Specifically, in each combination test group (a trametinib combination test group, a fluorouracil combination test group and an irinotecan combination test group), the indicated drug used in combination was applied to human cancer cells, and then the cancer cells were cocultured with human monocytic cells (THP-1) in the presence of the anti-CAPRIN-1 antibody, and the antibody-mediated phagocytic activity against the cancer cells by THP-1 was evaluated. The human-derived cancer cells used were melanoma cell line A375 (used only in the trametinib combination test group), colon cancer cell line HCT116, prostate cancer cell line DU145 (used only in the trametinib combination test group), and lung cancer cell line A549 (used only in the trametinib combination test group). On a 6-well plate, the human-derived cancer cells were cultured for 2 days in the presence of trametinib for the trametinib combination test group, and cultured for 2 days in the presence of the each drug used in combination for the fluorouracil combination test group and the irinotecan combination test group. The concentration of the drug used in combination was set to a concentration that decreased the proliferation rate of the cancer cells by about half at the completion of culture. Specifically, for HCT116, trametinib was added at a concentration of 50 nM, fluorouracil was added at a concentration of 0.4 µg/mL, and irinotecan was added at a concentration of 15 µM; trametinib was added to A375 at a concentration of 50 nM; trametinib was added to DU145 at a concentration of 250 nM; and trametinib was added to A549 at a concentration of 250 nM. As a control, a test group without a drug used in combination was established. In the control group, only DMSO (dimethyl sulfoxide), a solvent for the drug used in combination, was added to the cancer cells at a final concentration of 0.1%, and then the cancer cells were cultured for 2 days.

Each cancer cell line after the culture was dissociated with TrypLE Express (Thermo), and the cancer cells were stained by the addition of calcein-AM at a final concentration of 0.02 µg/mL and incubation at 37°C for 30 minutes. Next, the cells were dispensed at 1 × 10⁴ cancer cells per well to a 96-well plate, and the anti-CAPRIN-1 antibody (final concentration: 5 µg/mL) and 1 × 10⁵ THP-1 cells were added to each well, followed by culture at 37°C for 2.5 hours under a condition of 5% CO₂. Then, the cells were washed with PBS (phosphate buffer solution) containing 1% FBS (fetal bovine serum), and the human monocytic cells were stained through a reaction with an APC (allophycocyanin)-labeled anti-human CD45 antibody (final concentration: 0.25 µg/mL). Further, dead cells were stained with propidium iodide (PI) (final concentration: 0.1 µg/mL), followed by the measurement of fluorescence from the cells by flow cytometry. PI-positive dead cells were excluded from analysis. The human monocytic cells THP-1 recognize an antibody bound onto cancer cells and phagocytize the cancer cells. At that time, if the cancer cells have been stained with calcein-AM, the monocytic cells ingesting the cancer cells by phagocytosis also become positive to calcein-AM. Thus, the phagocytic activity in this evaluation system was calculated as the ratio (%) of APC-positive/calcein-AM-positive cells to the whole calcein-AM-positive population.

As a result of evaluation using the anti-CAPRIN-1 antibody prepared in Example 1 (anti-CAPRIN-1 humanized antibody #1) as an anti-CAPRIN-1 antibody, phagocytic activity against cancer cells of 18%, 11%, 4%, and 4% was observed for A375, HCT116, DU145, and A549, respectively, in the test group without a drug used in combination. By contrast, phagocytic activity against cancer cells of 49%, 45%, 9%, and 7% was observed for A375, HCT116, DU145, and A549, respectively, in the trametinib combination test group. Thus, the antitumor effect of the anti-CAPRIN-1 antibody on these four human cancer cells was increased by approximately 1.8 to 4 times by using the anti-CAPRIN-1 antibody in combination with trametinib (Figure 1).

For HCT116, phagocytic activity against cancer cells was 45% in the trametinib combination test group, whereas the phagocytic activity against cancer cells was 16% and 14% in the fluorouracil combination test group and the irinotecan combination test group, respectively. Thus, the combination of the anti-CAPRIN-1 antibody and trametinib strongly increased the phagocytic activity against cancer cells as compared with the combinations of the anti-CAPRIN-1 antibody and the existing chemotherapeutics described above (Figure 2).

Combinations of any anti-CAPRIN-1 antibody described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176 and WO2015/020212 and trametinib exhibit similar increase in phagocytic activity against the above human cancer cells as in the combination of the anti-CAPRIN-1 humanized antibody #1 and trametinib.

### (Example 3) In vitro antitumor effect of combination of anti-CAPRIN-1 antibody and RAS inhibitor

Antitumor effects of combinations of anti-CAPRIN-1 antibodies and RAS inhibitors were evaluated *in vitro.* The RAS inhibitors used were lonafarnib (Cat. No. S2797), sotorasib (Cat. No. S8830), and salirasib (Cat. No. S7684) purchased from Selleck Chemicals LLC.

Specifically, in each RAS inhibitor combination test group, the indicated drug used in combination was applied to human cancer cells, and then the cancer cells were cocultured with human monocytic cells (THP-1) in the presence of the anti-CAPRIN-1 antibody, and the antibody-mediated phagocytic activity against the cancer cells by THP-1 was evaluated.

The human-derived cancer cells used were a colon cancer cell line HCT116. On a 6-well plate, the human-derived cancer cells were cultured for 2 days in the presence of the indicated drug used in combination in the lonafarnib combination test group, the sotorasib combination test group and the salirasib combination test group. Specifically, for HCT116, lonafarnib was added at a concentration of 10 µM, sotorasib was added at a concentration of 10 µM, and salirasib was added at a concentration of 100 µM. As a control, a test group without a drug used in combination was established. In the control group, the cancer cells were cultured beforehand for 2 days in a state without addition of the drug used in combination.

Each cancer cell line after the culture was dissociated with TrypLE Express (Thermo), and the cancer cells were stained by the addition of calcein-AM at a final concentration of 0.04 µg/mL and incubation at 37°C for 10 minutes. Next, the cells were dispensed at 5 × 10³ cancer cells per well to a 96-well plate, and the anti-CAPRIN-1 antibody (final concentration: 1 µg/mL) and 1.25 × 10⁵ THP-1 cells were added to each well, followed by culture at 37°C for 1 hour under a condition of 5% CO₂. Then, the cells were washed with PBS (phosphate buffer solution) containing 1% FBS (fetal bovine serum), and the human monocytic cells were stained through a reaction with an APC (allophycocyanin)-labeled anti-human CD45 antibody (final concentration: 0.25 µg/mL). Further, dead cells were stained with propidium iodide (PI) (final concentration: 0.1 µg/mL), followed by the measurement of fluorescence from the cells by flow cytometry. PI-positive dead cells were excluded from analysis. The human monocytic cells THP-1 recognize an antibody bound onto cancer cells and phagocytize the cancer cells. At that time, if the cancer cells have been stained with calcein-AM, the monocytic cells ingesting the cancer cells by phagocytosis also become positive to calcein-AM. Thus, the phagocytic activity in this evaluation system was calculated as the ratio (%) of APC-positive/calcein-AM-positive cells to the whole calcein-AM-positive population.

As a result of evaluation using the anti-CAPRIN-1 antibody prepared in Example 1 (anti-CAPRIN-1 humanized antibody #1) as an anti-CAPRIN-1 antibody, phagocytic activity against cancer cells of 50% was observed for HCT116 in the test group without a drug used in combination. By contrast, phagocytic activity against cancer cells of 59% to 80% was observed for all the lonafarnib, sotorasib, and salirasib combination test groups. As a result of conducting Student's t test, the phagocytic activity of the lonafarnib, sotorasib, and salirasib combination test groups was significantly higher than that of the test group without a drug used in combination (p < 0.001, p < 0.01, and p < 0.001, respectively; significance level: 5%). Thus, the antitumor effect of the anti-CAPRIN-1 antibody on the human cancer cells was significantly increased by approximately 1.2 to 1.6 times by using the anti-CAPRIN-1 antibody in combination with lonafarnib, sotorasib, or salirasib (Figure 3).

Combinations of any anti-CAPRIN-1 antibody described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176 and WO2015/020212 and lonafarnib, sotorasib, or salirasib exhibit similar increase in phagocytic activity against human cancer cells as in the combination of the anti-CAPRIN-1 humanized antibody #1 and lonafarnib, sotorasib, or salirasib.

### (Example 4) In vitro antitumor effect of combination of anti-CAPRIN-1 antibody and RAF inhibitor

Antitumor effects of combinations of anti-CAPRIN-1 antibodies and RAF inhibitors were evaluated *in vitro.* The RAF inhibitors used were dabrafenib (Cat. No. CS-0692) and ZM 336372 (Cat. No. CS-0693) purchased from ChemScene LLC.

Specifically, in each RAF inhibitor combination test group, the indicated drug used in combination was applied to human cancer cells, and then the cancer cells were cocultured with human monocytic cells (THP-1) in the presence of the anti-CAPRIN-1 antibody, and the antibody-mediated phagocytic activity against the cancer cells by THP-1 was evaluated.

The human-derived cancer cells used were a colon cancer cell line HCT116. On a 6-well plate, the human-derived cancer cells were cultured for 2 days in the presence of the indicated drug used in combination in the dabrafenib combination test group and the ZM 336372 combination test group. Specifically, for HCT116,dabrafenib was added at a concentration of 10 µM and ZM 336372 was added at a concentration of 100 µM. As a control, a test group without a drug used in combination was established. In the control group, the cancer cells were cultured beforehand for 2 days in a state without addition of the drug used in combination.

Each cancer cell line after the culture was dissociated with TrypLE Express (Thermo), and the cancer cells were stained by the addition of calcein-AM at a final concentration of 0.04 µg/mL and incubation at 37°C for 10 minutes. Next, the cells were dispensed at 5 × 10³ cancer cells per well to a 96-well plate, and the anti-CAPRIN-1 antibody (final concentration: 1 µg/mL) and 1.25 × 10⁵ THP-1 cells were added to each well, followed by culture at 37°C for 1 hour under a condition of 5% CO₂. Then, the cells were washed with PBS (phosphate buffer solution) containing 1% FBS (fetal bovine serum), and the human monocytic cells were stained through a reaction with an APC (allophycocyanin)-labeled anti-human CD45 antibody (final concentration: 0.25 µg/mL). Further, dead cells were stained with propidium iodide (PI) (final concentration: 0.1 µg/mL), followed by the measurement of fluorescence from the cells by flow cytometry. PI-positive dead cells were excluded from analysis. The human monocytic cells THP-1 recognize an antibody bound onto cancer cells and phagocytize the cancer cells. At that time, if the cancer cells have been stained with calcein-AM, the monocytic cells ingesting the cancer cells by phagocytosis also become positive to calcein-AM. Thus, the phagocytic activity in this evaluation system was calculated as the ratio (%) of APC-positive/calcein-AM-positive cells to the whole calcein-AM-positive population.

As a result of evaluation using the anti-CAPRIN-1 antibody prepared in Example 1 (anti-CAPRIN-1 humanized antibody #1) as an anti-CAPRIN-1 antibody, phagocytic activity against cancer cells of 23% was observed for HCT116 in the test group without a drug used in combination. By contrast, phagocytic activity against cancer cells of 75% or more was observed in both the dabrafenib and ZM 336372 combination test groups. Thus, the antitumor effect of the anti-CAPRIN-1 antibody on the human cancer cells was increased by approximately 3.3 to 3.5 times by using the anti-CAPRIN-1 antibody in combination with dabrafenib or ZM 336372 (Figure 4).

Combinations of any anti-CAPRIN-1 antibody described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176 and WO2015/020212 and dabrafenib or ZM 336372 exhibit similar increase in phagocytic activity against human cancer cells as in the combination of the anti-CAPRIN-1 humanized antibody #1 and dabrafenib or ZM 336372.

### (Example 5) In vitro antitumor effect of combination of anti-CAPRIN-1 antibody and MEK inhibitor - 2

Antitumor effects of combinations of anti-CAPRIN-1 antibodies and MEK inhibitors other than trametinib were evaluated *in vitro.* The MEK inhibitors used were selumetinib (Cat. No. S1008), refametinib (Cat. No. S1089), and SL-327 (Cat. No. S1066) purchased from Selleck Chemicals LLC, and cobimetinib (Cat. No. 19563) purchased from Cayman Chemical Company.

Specifically, in each MEK inhibitor combination test group, the indicated drug used in combination was applied to human cancer cells, and then the cancer cells were cocultured with human monocytic cells (THP-1) in the presence of the anti-CAPRIN-1 antibody, and the antibody-mediated phagocytic activity against the cancer cells by THP-1 was evaluated.

The human-derived cancer cells used were a colon cancer cell line HCT116. On a 6-well plate, the human-derived cancer cells were cultured for 2 days in the presence of the indicated drug used in combination in the selumetinib combination test group, the refametinib combination test group, the SL-327 combination test group and the cobimetinib bosutinib combination test group. Specifically, for HCT116, selumetinib was added at a concentration of 10 µM, refametinib was added at a concentration of 1000 nM, SL-327 was added at a concentration of 10 µM, and cobimetinib was added at a concentration of 800 nM. As a control, a test group without a drug used in combination was established. In the control group, the cancer cells were cultured beforehand for 2 days in a state without addition of the drug used in combination.

Each cancer cell line after the culture was dissociated with TrypLE Express (Thermo), and the cancer cells were stained by the addition of calcein-AM at a final concentration of 0.04 µg/mL and incubation at 37°C for 10 minutes. Next, the cells were dispensed at 5 × 10³ cancer cells per well to a 96-well plate, and the anti-CAPRIN-1 antibody (final concentration: 1 µg/mL) and 1.25 × 10⁵ THP-1 cells were added to each well, followed by culture at 37°C for 1 hour under a condition of 5% CO₂. Then, the cells were washed with PBS (phosphate buffer solution) containing 1% FBS (fetal bovine serum), and the human monocytic cells were stained through a reaction with an APC (allophycocyanin)-labeled anti-human CD45 antibody (final concentration: 0.25 µg/mL). Further, dead cells were stained with propidium iodide (PI) (final concentration: 0.1 µg/mL), followed by the measurement of fluorescence from the cells by flow cytometry. PI-positive dead cells were excluded from analysis. The human monocytic cells THP-1 recognize an antibody bound onto cancer cells and phagocytize the cancer cells. At that time, if the cancer cells have been stained with calcein-AM, the monocytic cells ingesting the cancer cells by phagocytosis also become positive to calcein-AM. Thus, the phagocytic activity in this evaluation system was calculated as the ratio (%) of APC-positive/calcein-AM-positive cells to the whole calcein-AM-positive population.

As a result of evaluation using the anti-CAPRIN-1 antibody prepared in Example 1 (anti-CAPRIN-1 humanized antibody #1) as an anti-CAPRIN-1 antibody, phagocytic activity against cancer cells of 50% was observed for HCT116 in the c test group without a drug used in combination. By contrast, phagocytic activity against cancer cells of 93% or more was observed for all the selumetinib, refametinib, SL-327, and cobimetinib combination test groups. Thus, the antitumor effect of the anti-CAPRIN-1 antibody on the human cancer cells was significantly increased by 1.8 times by using the anti-CAPRIN-1 antibody in combination with selumetinib, refametinib, SL-327, or cobimetinib (Figure 3).

Combinations of any anti-CAPRIN-1 antibody described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176 and WO2015/020212 and selumetinib, refametinib, SL-327, or cobimetinib exhibit similar increase in phagocytic activity against human cancer cells as in the combination of the anti-CAPRIN-1 humanized antibody #1 and selumetinib, refametinib, SL-327, or cobimetinib.

### (Example 6) In vitro antitumor effect of combination of anti-CAPRIN-1 antibody and ERK inhibitor

Antitumor effects of combinations of anti-CAPRIN-1 antibodies and ERK inhibitors were evaluated *in vitro.* The ERK inhibitors used were bosutinib(Cat. No. S 1014) purchased from Selleck Chemicals LLC and SCH772984 (Cat. No. 19166) purchased from Cayman Chemical Company.

Specifically, in each ERK inhibitor combination test group, the indicated drug used in combination was applied to human cancer cells, and then the cancer cells were cocultured with human monocytic cells (THP-1) in the presence of the anti-CAPRIN-1 antibody, and the antibody-mediated phagocytic activity against the cancer cells by THP-1 was evaluated.

The human-derived cancer cells used were a colon cancer cell line HCT116. On a 6-well plate, the human-derived cancer cells were cultured for 2 days in the presence of the indicated drug used in combination in the SCH772984 combination test group and the bosutinib combination test group. Specifically, for HCT116, SCH772984 was added at a concentration of 10 µM and bosutinib was added at a concentration of 10 µM. As a control, a test group without a drug used in combination was established. In the control group, and the cancer cells were cultured beforehand for 2 days in a state without addition of the drug used in combination.

Each cancer cell line after the culture was dissociated with TrypLE Express (Thermo), and the cancer cells were stained by the addition of calcein-AM at a final concentration of 0.04 µg/mL and incubation at 37°C for 10 minutes. Next, the cells were dispensed at 5 × 10³ cancer cells per well to a 96-well plate, and the anti-CAPRIN-1 antibody (final concentration: 1 µg/mL) and 1.25 × 10⁵ THP-1 cells were added to each well, followed by culture at 37°C for 1 hour under a condition of 5% CO₂. Then, the cells were washed with PBS (phosphate buffer solution) containing 1% FBS (fetal bovine serum), and the human monocytic cells were stained through a reaction with an APC (allophycocyanin)-labeled anti-human CD45 antibody (final concentration: 0.25 µg/mL). Further, dead cells were stained with propidium iodide (PI) (final concentration: 0.1 µg/mL), followed by the measurement of fluorescence from the cells by flow cytometry. PI-positive dead cells were excluded from analysis. The human monocytic cells THP-1 recognize an antibody bound onto cancer cells and phagocytize the cancer cells. At that time, if the cancer cells have been stained with calcein-AM, the monocytic cells ingesting the cancer cells by phagocytosis also become positive to calcein-AM. Thus, the phagocytic activity in this evaluation system was calculated as the ratio (%) of APC-positive/calcein-AM-positive cells to the whole calcein-AM-positive population.

As a result of evaluation using the anti-CAPRIN-1 antibody prepared in Example 1 (anti-CAPRIN-1 humanized antibody #1) as an anti-CAPRIN-1 antibody, phagocytic activity against cancer cells of 39% was observed for HCT116 in the test group without a drug used in combination. By contrast, phagocytic activity against cancer cells of 90% was observed for the SCH772984 combination test group (Figure 5). As a result of conducting Student's t test, the phagocytic activity of the SCH772984 combination test group was significantly higher than that of the test group without a drug used in combination (p < 0.001; significance level: 5%). Also, phagocytic activity against cancer cells of 50% was observed for HCT116 in the test group without a drug used in combination. By contrast, phagocytic activity against cancer cells of 82% was observed in the bosutinib combination test group (Figure 3). As a result of conducting Student's t test, the phagocytic activity of the bosutinib combination test group was significantly higher than that of the test group without a drug used in combination (p < 0.001; significance level: 5%). Thus, the antitumor effect of the anti-CAPRIN-1 antibody on the human cancer cells was significantly increased by approximately 1.7 to 2.3 times by using the anti-CAPRIN-1 antibody in combination with bosutinib or SCH772984.

Combinations of any anti-CAPRIN-1 antibody described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176 and WO2015/020212 and bosutinib or SCH772984 exhibit similar increase in phagocytic activity against human cancer cells as in the combination of the anti-CAPRIN-1 humanized antibody #1 and bosutinib or SCH772984.

All publications, patents and patent applications cited herein are incorporated herein by reference in their entirety.

## Claims

1. A medicament for treatment and/or prevention of cancer, comprising an antibody or a fragment thereof having an immunological reactivity with CAPRIN-1 protein, and a MAPK (mitogen-activated protein kinase) pathway inhibitor together or separately in combination.

2. The medicament according to claim 1, wherein the MAPK pathway inhibitor comprises a MEK inhibitor, a RAS inhibitor, a RAF inhibitor, an ERK inhibitor, or a combination of any two or more thereof.

3. The medicament according to claim 2, wherein the MEK inhibitor is at least one selected from the group consisting of trametinib, cobimetinib, selumetinib, refametinib, SL-327, and their derivatives.

4. The medicament according to claim 2, wherein the RAS inhibitor is at least one selected from the group consisting of sotorasib, lonafarnib, salirasib, and their derivatives.

5. The medicament according to claim 2, wherein the RAF inhibitor is at least one selected from the group consisting of dabrafenib, ZM 336372, and their derivatives.

6. The medicament according to claim 2, wherein the ERK inhibitor is at least one selected from the group consisting of bosutinib, SCH772984, and their derivatives.

7. The medicament according to any one of claims 1 to 6, wherein the antibody or the fragment thereof has an immunological reactivity with CAPRIN-1 protein having an amino acid sequence shown by any one of the even numbered SEQ ID NOs: 2 to 30, or an amino acid sequence having 80% or more sequence identity with the amino acid sequence.

8. The medicament according to any one of claims 1 to 7, wherein the antibody or the fragment thereof has an immunological reactivity with an extracellular region of CAPRIN-1 protein present on a cancer cell surface.

9. The medicament according to any one of claims 1 to 8, wherein the antibody or the fragment thereof has an immunological reactivity with a partial polypeptide of CAPRIN-1 protein, the partial polypeptide having an amino acid sequence shown by any one of SEQ ID NOs: 31 to 35, 296 to 299, 308 and 309, or an amino acid sequence having 80% or more sequence identity with the amino acid sequence.

10. The medicament according to any one of claims 1 to 9, wherein the antibody is a monoclonal antibody or a polyclonal antibody.

11. The medicament according to any one of claims 1 to 10, wherein the antibody or the fragment thereof is any one of the following (A) to (M):
(A) an antibody or a fragment thereof comprising a heavy-chain variable region comprising complementarity determining regions of SEQ ID NOs: 36, 37 and 38 (CDR1, CDR2 and CDR3, respectively) and a light-chain variable region comprising complementarity determining regions of SEQ ID NOs: 40, 41 and 42 (CDR1, CDR2 and CDR3, respectively), and having an immunological reactivity with CAPRIN-1 protein;
(B) an antibody or a fragment thereof comprising a heavy-chain variable region comprising complementarity determining regions of SEQ ID NOs: 44, 45 and 46 (CDR1, CDR2 and CDR3, respectively) and a light-chain variable region comprising complementarity determining regions of SEQ ID NOs: 48, 49 and 50 (CDR1, CDR2 and CDR3, respectively), and having an immunological reactivity with CAPRIN-1 protein;
(C) an antibody or a fragment thereof comprising a heavy-chain variable region comprising complementarity determining regions of SEQ ID NOs: 52, 53 and 54 (CDR1, CDR2 and CDR3, respectively) and a light-chain variable region comprising complementarity determining regions of SEQ ID NOs: 56, 57 and 58 (CDR1, CDR2 and CDR3, respectively), and having an immunological reactivity with CAPRIN-1 protein;
(D) an antibody or a fragment thereof comprising a heavy-chain variable region comprising complementarity determining regions of SEQ ID NOs: 60, 61 and 62 (CDR1, CDR2 and CDR3, respectively) and a light-chain variable region comprising complementarity determining regions of SEQ ID NOs: 64, 65 and 66 (CDR1, CDR2 and CDR3, respectively), and having an immunological reactivity with CAPRIN-1 protein;
(E) an antibody or a fragment thereof comprising a heavy-chain variable region comprising complementarity determining regions of SEQ ID NOs: 170, 171 and 172 (CDR1, CDR2 and CDR3, respectively) and a light-chain variable region comprising complementarity determining regions of SEQ ID NOs: 173, 174 and 175 (CDR1, CDR2 and CDR3, respectively), and having an immunological reactivity with CAPRIN-1 protein;
(F) an antibody or a fragment thereof comprising a heavy-chain variable region comprising complementarity determining regions of SEQ ID NOs: 176, 177 and 178 (CDR1, CDR2 and CDR3, respectively) and a light-chain variable region comprising complementarity determining regions of SEQ ID NOs: 179, 180 and 181 (CDR1, CDR2 and CDR3, respectively), and having an immunological reactivity with CAPRIN-1 protein;
(G) an antibody or a fragment thereof comprising a heavy-chain variable region comprising complementarity determining regions of SEQ ID NOs: 182, 183 and 184 (CDR1, CDR2 and CDR3, respectively) and a light-chain variable region comprising complementarity determining regions of SEQ ID NOs: 185, 186 and 187 (CDR1, CDR2 and CDR3, respectively), and having an immunological reactivity with CAPRIN-1 protein;
(H) an antibody or a fragment thereof comprising a heavy-chain variable region comprising complementarity determining regions of SEQ ID NOs: 188, 189 and 190 (CDR1, CDR2 and CDR3, respectively) and a light-chain variable region comprising complementarity determining regions of SEQ ID NOs: 191, 192 and 193 (CDR1, CDR2 and CDR3, respectively), and having an immunological reactivity with CAPRIN-1 protein;
(I) an antibody or a fragment thereof comprising a heavy-chain variable region comprising complementarity determining regions of SEQ ID NOs: 146, 147 and 148 (CDR1, CDR2 and CDR3, respectively) and a light-chain variable region comprising complementarity determining regions of SEQ ID NOs: 149, 150 and 151 (CDR1, CDR2 and CDR3, respectively), and having an immunological reactivity with CAPRIN-1 protein;
(J) an antibody or a fragment thereof comprising a heavy-chain variable region comprising complementarity determining regions of SEQ ID NOs: 272, 273 and 274 (CDR1, CDR2 and CDR3, respectively) and a light-chain variable region comprising complementarity determining regions of SEQ ID NOs: 275, 276 and 277 (CDR1, CDR2 and CDR3, respectively), and having an immunological reactivity with CAPRIN-1 protein;
(K) an antibody or a fragment thereof comprising a heavy-chain variable region comprising complementarity determining regions of SEQ ID NOs: 290, 291 and 292 (CDR1, CDR2 and CDR3, respectively) and a light-chain variable region comprising complementarity determining regions of SEQ ID NOs: 293, 294 and 295 (CDR1, CDR2 and CDR3, respectively), and having an immunological reactivity with CAPRIN-1 protein;
(L) an antibody or a fragment thereof comprising a heavy-chain variable region comprising complementarity determining regions of SEQ ID NOs: 301, 302 and 303 (CDR1, CDR2 and CDR3, respectively) and a light-chain variable region comprising complementarity determining regions of SEQ ID NOs: 305, 306 and 307 (CDR1, CDR2 and CDR3, respectively), and having an immunological reactivity with CAPRIN-1 protein; and
(M) an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein.

12. The medicament according to any one of claims 1 to 11, wherein the antibody or the fragment thereof is any one of the following (a) to (al):
(a) an antibody or a fragment thereof comprising a heavy-chain variable region comprising the amino acid sequence of SEQ ID NO: 39 and a light-chain variable region comprising the amino acid sequence of SEQ ID NO: 43;
(b) an antibody or a fragment thereof comprising a heavy-chain variable region comprising the amino acid sequence of SEQ ID NO: 47 and a light-chain variable region comprising the amino acid sequence of SEQ ID NO: 51;
(c) an antibody or a fragment thereof comprising a heavy-chain variable region comprising the amino acid sequence of SEQ ID NO: 55 and a light-chain variable region comprising the amino acid sequence of SEQ ID NO: 59;
(d) an antibody or a fragment thereof comprising a heavy-chain variable region comprising the amino acid sequence of SEQ ID NO: 63 and a light-chain variable region comprising the amino acid sequence of SEQ ID NO: 67;
(e) an antibody or a fragment thereof comprising a heavy-chain variable region comprising the amino acid sequence of SEQ ID NO: 68 and a light-chain variable region comprising the amino acid sequence of SEQ ID NO: 69;
(f) an antibody or a fragment thereof comprising a heavy-chain variable region comprising the amino acid sequence of SEQ ID NO: 70 and a light-chain variable region comprising the amino acid sequence of SEQ ID NO: 71;
(g) an antibody or a fragment thereof comprising a heavy-chain variable region comprising the amino acid sequence of SEQ ID NO: 72 and a light-chain variable region comprising the amino acid sequence of SEQ ID NO: 73;
(h) an antibody or a fragment thereof comprising a heavy-chain variable region comprising the amino acid sequence of SEQ ID NO: 74 and a light-chain variable region comprising the amino acid sequence of SEQ ID NO: 75;
(i) an antibody or a fragment thereof comprising a heavy-chain variable region comprising the amino acid sequence of SEQ ID NO: 76 and a light-chain variable region comprising the amino acid sequence of SEQ ID NO: 77;
(j) an antibody or a fragment thereof comprising a heavy-chain variable region comprising the amino acid sequence of SEQ ID NO: 78 and a light-chain variable region comprising the amino acid sequence of SEQ ID NO: 79;
(k) an antibody or a fragment thereof comprising a heavy-chain variable region comprising the amino acid sequence of SEQ ID NO: 80 and a light-chain variable region comprising the amino acid sequence of SEQ ID NO: 81;
(l) an antibody or a fragment thereof comprising a heavy-chain variable region comprising the amino acid sequence of SEQ ID NO: 82 and a light-chain variable region comprising the amino acid sequence of SEQ ID NO: 83;
(m) an antibody or a fragment thereof comprising a heavy-chain variable region comprising the amino acid sequence of SEQ ID NO: 84 and a light-chain variable region comprising the amino acid sequence of SEQ ID NO: 85;
(n) an antibody or a fragment thereof comprising a heavy-chain variable region comprising the amino acid sequence of SEQ ID NO: 86 and a light-chain variable region comprising the amino acid sequence of SEQ ID NO: 87;
(o) an antibody or a fragment thereof comprising a heavy-chain variable region comprising the amino acid sequence of SEQ ID NO: 88 and a light-chain variable region comprising the amino acid sequence of SEQ ID NO: 89;
(p) an antibody or a fragment thereof comprising a heavy-chain variable region comprising the amino acid sequence of SEQ ID NO: 90 and a light-chain variable region comprising the amino acid sequence of SEQ ID NO: 91;
(q) an antibody or a fragment thereof comprising a heavy-chain variable region comprising the amino acid sequence of SEQ ID NO: 92 and a light-chain variable region comprising the amino acid sequence of SEQ ID NO: 93;
(r) an antibody or a fragment thereof comprising a heavy-chain variable region comprising the amino acid sequence of SEQ ID NO: 94 and a light-chain variable region comprising the amino acid sequence of SEQ ID NO: 95;
(s) an antibody or a fragment thereof comprising a heavy-chain variable region comprising the amino acid sequence of SEQ ID NO: 96 and a light-chain variable region comprising the amino acid sequence of SEQ ID NO: 97;
(t) an antibody or a fragment thereof comprising a heavy-chain variable region comprising the amino acid sequence of SEQ ID NO: 98 and a light-chain variable region comprising the amino acid sequence of SEQ ID NO: 99;
(u) an antibody or a fragment thereof comprising a heavy-chain variable region comprising the amino acid sequence of SEQ ID NO: 100 and a light-chain variable region comprising the amino acid sequence of SEQ ID NO: 101;
(v) an antibody or a fragment thereof comprising a heavy-chain variable region comprising the amino acid sequence of SEQ ID NO: 102 and a light-chain variable region comprising the amino acid sequence of SEQ ID NO: 103;
(w) an antibody or a fragment thereof comprising a heavy-chain variable region comprising the amino acid sequence of SEQ ID NO: 104 and a light-chain variable region comprising the amino acid sequence of SEQ ID NO: 105;
(x) an antibody or a fragment thereof comprising a heavy-chain variable region comprising the amino acid sequence of SEQ ID NO: 106 and a light-chain variable region comprising the amino acid sequence of SEQ ID NO: 107;
(y) an antibody or a fragment thereof comprising a heavy-chain variable region comprising the amino acid sequence of SEQ ID NO: 108 and a light-chain variable region comprising the amino acid sequence of SEQ ID NO: 109;
(z) an antibody or a fragment thereof comprising a heavy-chain variable region comprising the amino acid sequence of SEQ ID NO: 110 and a light-chain variable region comprising the amino acid sequence of SEQ ID NO: 111;
(aa) an antibody or a fragment thereof comprising a heavy-chain variable region comprising the amino acid sequence of SEQ ID NO: 112 and a light-chain variable region comprising the amino acid sequence of SEQ ID NO: 113;
(ab) an antibody or a fragment thereof comprising a heavy-chain variable region comprising the amino acid sequence of SEQ ID NO: 114 and a light-chain variable region comprising the amino acid sequence of SEQ ID NO: 115;
(ac) an antibody or a fragment thereof comprising a heavy-chain variable region comprising the amino acid sequence of SEQ ID NO: 116 and a light-chain variable region comprising the amino acid sequence of SEQ ID NO: 117;
(ad) an antibody or a fragment thereof comprising a heavy-chain variable region comprising the amino acid sequence of SEQ ID NO: 118 and a light-chain variable region comprising the amino acid sequence of SEQ ID NO: 119;
(ae) an antibody or a fragment thereof comprising a heavy-chain variable region comprising the amino acid sequence of SEQ ID NO: 120 and a light-chain variable region comprising the amino acid sequence of SEQ ID NO: 121;
(af) an antibody or a fragment thereof comprising a heavy-chain variable region comprising the amino acid sequence of SEQ ID NO: 122 and a light-chain variable region comprising the amino acid sequence of SEQ ID NO: 123;
(ag) an antibody or a fragment thereof comprising a heavy-chain variable region comprising the amino acid sequence of SEQ ID NO: 124 and a light-chain variable region comprising the amino acid sequence of SEQ ID NO: 125;
(ah) an antibody or a fragment thereof comprising a heavy-chain variable region comprising the amino acid sequence of SEQ ID NO: 126 and a light-chain variable region comprising the amino acid sequence of SEQ ID NO: 127;
(ai) an antibody or a fragment thereof comprising a heavy-chain variable region comprising the amino acid sequence of SEQ ID NO: 128 and a light-chain variable region comprising the amino acid sequence of SEQ ID NO: 129;
(aj) an antibody or a fragment thereof comprising a heavy-chain variable region comprising the amino acid sequence of SEQ ID NO: 130 and a light-chain variable region comprising the amino acid sequence of SEQ ID NO: 131;
(ak) an antibody or a fragment thereof comprising a heavy-chain variable region comprising the amino acid sequence of SEQ ID NO: 132 and a light-chain variable region comprising the amino acid sequence of SEQ ID NO: 133; and
(al) an antibody or a fragment thereof comprising a heavy-chain variable region comprising the amino acid sequence of SEQ ID NO: 300 and a light-chain variable region comprising the amino acid sequence of SEQ ID NO: 304.

13. The medicament according to any one of claims 1 to 12, wherein the antibody is a human antibody, a humanized antibody, a chimeric antibody or a single chain antibody.

14. The medicament according to any one of claims 1 to 13, wherein the cancer is a cancer expressing CAPRIN-1 protein on a cell membrane surface.

15. The medicament according to any one of claims 1 to 14, wherein the cancer is melanoma, lung cancer, thyroid cancer, colon cancer, prostate cancer, ovarian cancer, pancreatic cancer, kidney cancer, breast cancer, gastric cancer, bile duct cancer, renal cell cancer, Hodgkin's lymphoma, head and neck cancer, mesothelioma, colorectal cancer, esophageal cancer, gastroesophageal cancer, hepatocellular cancer, glioblastoma, urothelial cancer, urinary bladder cancer, uterus cancer, primary central nervous system lymphoma, primary testicular lymphoma, biliary tract cancer, brain tumor, leukemia, lymphoma, liver cancer, sarcoma, fibrosarcoma, mastocytoma, adrenal cortex cancer, Ewing's tumor, multiple myeloma, testicle cancer, basal cell cancer, Paget's disease or skin cancer.

16. An agent increasing drug efficacy of a pharmaceutical composition for treatment and/or prevention of cancer comprising an antibody or a fragment thereof having an immunological reactivity with CAPRIN-1 protein as an active ingredient, wherein the agent comprises a MAPK pathway inhibitor as an active ingredient.

17. An agent increasing drug efficacy of a pharmaceutical composition for treatment and/or prevention of cancer comprising a MAPK pathway inhibitor as an active ingredient, wherein the agent comprises an antibody or a fragment thereof having an immunological reactivity with CAPRIN-1 protein as an active ingredient.

18. A method for treating and/or preventing cancer, comprising administering an antibody or a fragment thereof having an immunological reactivity with CAPRIN-1 protein, and a MAPK pathway inhibitor together or separately to a subject.
